# EUROPEAN PATENT APPLICATION

(11) **EP 4 148 058 A1**
(43) Date of publication of application: **15.03.2023**
(21) Application number: 21306257.3
(22) Date of filing: 13.09.2021
(51) Int. Cl.: C07D 519/00, A61B 5/00, A61K 49/00, G01N 33/00, G01R 33/00

(54) **TRITYL-NITROXIDE MULTIRADICALS AS POLARIZING AGENTS FOR DYNAMIC NUCLEAR POLARIZATION**

(71) Applicant: Commissariat à l'Energie Atomique et aux Energies Alternatives, 75015 Paris (FR); University of Iceland, 101 Reykjavik (IS); Université Grenoble Alpes, 38400 Saint-Martin-d'Hères (FR)
(72) Inventor: DE PAËPE, Gaël, 38000 GRENOBLE (FR); HARRABI, Rania, 38000 GRENOBLE (FR); MENTINK-VIGIER, Frédéric, 30220 Aigues Mortes (FR); SIGURDSSON, Snorri Th., Reykjavik (IS); HALBRITTER, Thomas, Reykjanesbaer (IS)
(74) Representative: Gevers & Orès

(57) **Abstract**

The present invention relates to novel trityl-nitroxide radicals as polarizing agents for Dynamic Nuclear Polarization (DNP).

## Description

### FIELD OF THE INVENTION

The present invention relates to the field of Dynamic Nuclear Polarization (DNP), in particular to novel trityl-nitroxide radicals as polarizing agents for Dynamic Nuclear Polarization (DNP).

### BACKGROUND

Nuclear magnetic resonance (NMR) is a very informative characterization method that allows probing matter by detecting signals of nuclear spins placed in a sufficiently stable, homogeneous and intense magnetic field, typically up to tens of Tesla (T). This technique makes it possible to record spectral data, which, once interpreted, gives information at the atomic scale on the structure, and the dynamics of the system studied. The use of NMR is widespread in chemistry, biology and materials science.

When one wishes to study a sample in the solid-state, an approach based mainly on pneumatic and rapid rotation of the sample at the magic angle is used. This technique, called "magic angle spinning" (MAS), significantly improves the spectral resolution. This method provides extensive information but suffers from a lack of sensitivity in comparison with other analytical techniques. The acquisition of signals of interest is therefore often long and/or requires the use of a sufficiently large amount of sample (1 to 100 mg typically). This limitation is inherent to the properties of nuclei and is directly related to the low polarization of nuclear spin transitions. This lack of sensitivity undoubtedly limits the use of NMR for the study and development of new functional materials (e.g. catalysis, energy storage), as well as for the studies of complex biomolecular systems (e.g. globular, membrane, and fibrillar proteins).

To compensate for this lack of sensitivity, a technique of hyperpolarization, called Dynamic Nuclear Polarization (DNP), can be used. This phenomenon, discovered in 1953, for weak magnetic fields, has become a field with strong development since 2009, after the work from Professor Griffin's group at MIT (D. A. Hall et al., Science (80-), vol. 276, no. 5314, pp. 930-932, 1997, doi: 10.1126/science.276.5314.930; A. B. Barnes et al., Appl. Magn. Reson., vol. 34, no. 3-4, pp. 237-263, 2008, doi: 10.1007/s00723-008-0129-1; R. Griffin et al., Phys. Chem. Chem. Phys, vol. 12, p. 5850, 2010). Thanks to major instrumental developments, including access to high-power and high frequency microwave sources, DNP applied to solid-state nuclear magnetic resonance in rotation at the magic angle, appears as a promising solution for significantly increasing nuclear magnetization in many systems. The DNP measurements are currently typically performed at magnetic fields of about 1 to 21.1 T, and in most cases require measurements at low temperature (about 100 K). The measurements are carried out in sample holders (rotors) with a capacity of 0.3 to 40 µL (D. Lee, S. Hediger, and G. De Paëpe, Solid State Nucl. Magn. Reson., vol. 66-67, pp. 6-20, Apr. 2015, doi: 10.1016/j.ssnmr.2015.01.003).

In high magnetic field DNP experiments, the systems of interest are generally dissolved, suspended or impregnated with a solution containing polarizing agents (PA). This solution is typically chosen depending on the chemical compatibility with the system to be studied, and for its characteristics in terms of DNP efficiency (quality of the glass formed at low temperature, relaxation time of nuclear and electronic spins). The polarizing agents contain paramagnetic centers (with unpaired electrons), which give them an electron spin, about 658 times more polarized than a nuclear proton spin. The application of a suitable microwave irradiation makes it possible to transfer the magnetization of the electronic spins to the surrounding nuclear spins and, thus, to significantly increase the detected NMR signal. Numerous polarizing agents have been developed over the years in order to optimize the signal-to-noise amplification by DNP.

Several magnetization transfer mechanisms have been described so far, such as "Solid-Effect" (SE) and "Cross-Effect" (CE). These two effects have recently been described theoretically under sample spinning and high magnetic field conditions (K. R. Thurber and R. Tycko, J. Chem. Phys., vol. 137, no. 8, p. 084508, Aug. 2012, doi: 10.1063/1.4747449; F. Mentink-Vigier et al., J. Magn. Reson., vol. 224, pp. 13-21, Nov. 2012, doi: 10.1016/j.jmr.2012.08.013).

The so-called "Cross-Effect" (CE) is one of the most efficient approach for DNP under "magic angle spinning" (MAS-DNP). It is typically obtained using tailored made biradicals as polarizing agents. Efficient polarizing agents maximize the polarization transfer while minimizing the time for such transfer under the most general experimental conditions: in the presence of a strong magnetic field, and/or rapid rotation of the sample.

Under the most advanced conditions in MAS-DNP (in 2021), that is to say a magnetic field of about 5 to 21.1 T or more, a sample rotation frequency in the 1 to 65 kHz range (or more) and a temperature of about 100 K, the CE is indeed currently the method that offers the best results for MAS-DNP experiments. This type of experiment involves the use of PAs that typically contain two paramagnetic centers, for example, two nitroxide entities connected by a bridge. This bridge, which ensures a substantial interaction between the electronic spins, is necessary for the CE mechanism. This interaction (dipolar or *J*-exchange) must be significant but not too large compared to the Larmor Frequency of the nuclei since it will decrease the CE efficiency.

The coupling between the electron spins is not the only important criterion for optimizing the polarization gain. Other interactions such as the *g*-tensors of each spin, which represent the coupling between the electron spins and its effective magnetic field environment, are also important. For instance, in the case of bi-nitroxide biradicals, the relative orientation of the two *g*-tensors has an influence on the CE efficiency, and one should avoid situations when the two *g*-tensors are parallel. Additionally, electron spin relaxation times (T1e/T2e), hyperfine couplings from the electron spins to the surrounding nuclei, as well as nuclear relaxation times are also important parameters with a strong impact on the CE efficiency. Ultimately, it is a complex set of parameters that governs the effectiveness of a DNP experiment. The molecules most commonly used correspond to bi-nitroxide biradicals (Totapol, the bTbK family, the bTUrea family) (C. Song et al., J. Am. Chem. Soc., vol. 128, no. 35, pp. 11385-90, Sep. 2006, doi: 10.1021/ja061284b; Y. Matsuki et al., Angew. Chem. Int. Ed. Engl., vol. 48, no. 27, pp. 4996-5000, Jan. 2009, doi: 10.1002/anie.200805940; C. Sauvee et al., Chem. - A Eur. J., vol. 22, no. 16, pp. 5598-5606, Apr. 2016, doi: 10.1002/chem.201504693), or hetero-biradicals (Tempo-Trityl and Tempo-BDPA) (G. Mathies et al., Angew. Chemie - Int. Ed., vol. 54, no. 40, pp. 11770-11774, 2015, doi: 10.1002/anie.201504292; D. Wisser et al., J. Am. Chem. Soc., vol. 140, no. 41, pp. 13340-13349, 2018, doi: 10.1021/jacs.8b08081; P. Berruyer et al., J. Phys. Chem. Lett., vol. 11, no. 19, pp. 8386-8391, 2020, doi: 10.1021/acs.jpclett.0c02493.

From the perspectives of developing biradicals, one of the main problems is to find a chemical bridge, as well as chemical substituents that can provide both a sufficient interaction between the electronic spins, a favorable relative position and orientation of the g-tensors, and interesting relaxation properties while maintaining good solubility in a DNP-compatible solvent suitable with the envisioned NMR application. Furthermore, the synthesis of the polarizing agents needs to be compatible with gram-scale synthesis.

The PAs proposed up to now work reasonably well with high-power (about 1 to 10 W) high-frequency microwave sources (e.g. gyrotrons) (R. Griffin et al., Phys. Chem. Chem. Phys, vol. 12, p. 5850, 2010) for magnetic fields up to about 15-20 T, and MAS frequencies up to 30-65 kHz. Nevertheless, there is still much room for improvement and there is thus still a need to pursue the development of PAs efficient, especially at very high field (> 10-15 T) and ultra-fast MAS (> 30 kHz). In addition to this first challenge, there is also a need to develop new PAs that are efficient when using low power microwave irradiation (10 mW to 1 W). This is particularly true for magnetic fields of up to 10 T, for which alternative microwave sources to the gyrotrons have already been demonstrated but with reduced DNP efficiency (I. V. Sergeyev et al., Solid State Nucl. Magn. Reson., vol. 100, no. February, pp. 63-69, 2019, doi: 10.1016/j.ssnmr.2019.03.008). The main advantages of these sources are their reduced cost and the limited laboratory space required for their installation. The development of suitable PAs thus holds the promise to successfully extend the DNP market to many NMR facilities (academic and private). In addition, such low power sources do not operate at a fixed frequency (unlike gyrotron devices currently installed in DNP laboratories). The microwave frequency can thus be set to maximize the DNP efficiency for each polarizing agent, without sweeping the NMR magnet (unlike frequency-fixed gyrotron). This implies that the NMR magnet does not need to be sweepable which further reduces the overall cost of a DNP setup equipment.

It is thus of high interest to develop new PAs with enhanced polarization transfer and minimized time for such transfer.

Furthermore, there is a real need for new PAs that can provide both a sufficient interaction between the electronic spins, a favorable relative position and orientation of the g-tensors, and interesting relaxation properties, while maintaining good solubility in a DNP-compatible solvent suitable for the intended application.

In particular, there is a need for new polarizing agents,
- that are efficient at very high field (>10-15 T), and/or
- that are efficient at ultra-fast MAS (> 30 kHz), and/or
- that are efficient with high and low power microwave irradiation (10 mW to 5 W or more).
- that can be produced in large quantities
- that can be dissolved easily in aqueous or organic solvents suitable for DNP experiments.

### SUMMARY OF THE INVENTION

The present invention addresses these needs among others by providing a compound of formula (I) 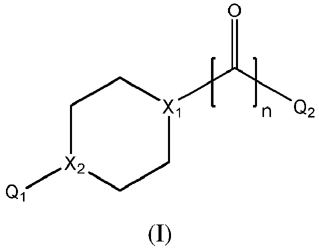 wherein
- n is 0 or 1;
- X₁ and X₂ are, independently, N, P, S;
- Q₂ is 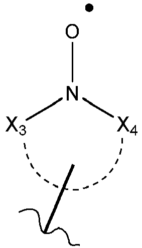 wherein
   - X₃ and X₄ are joined, as indicated by 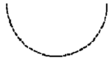 to form together with the nitrogen atom to which they are bound a 5- to 8-membered heterocyclic ring, the heterocyclic ring being optionally substituted, and/or
      the heterocyclic ring optionally containing one or more carbon-carbon double bond,
   - 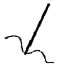 is the point of attachment of the 5- to 8-membered heterocyclic ring to the rest of the molecule;
- Q₁ is selected from 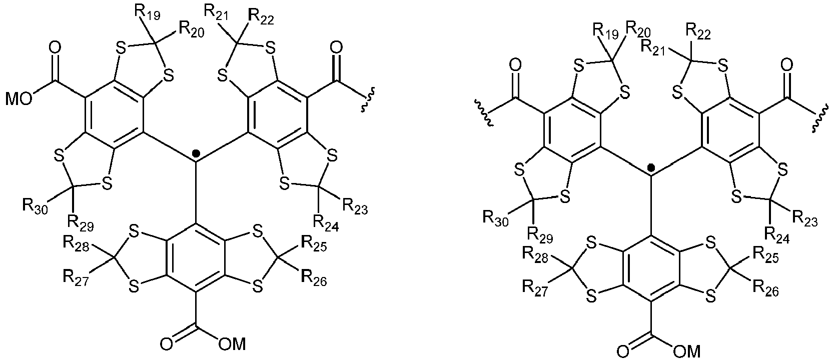 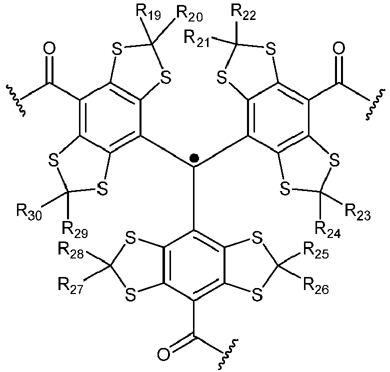 wherein
   - R₁₉ to R₃₀ are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms;
   - M is a hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms, an alkali cation selected from Na⁺ or K⁺, an ammonium cation (NH₄)⁺;
      with the proviso that the compound of formula 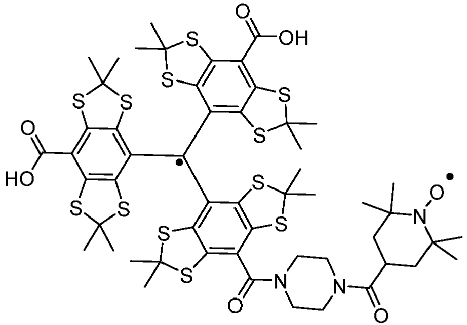 is excluded.

The compounds of formula (I) are highly efficient trityl-nitroxide radicals where the bridge/linker between Q₁ (the trityl moiety) and Q₂ (the nitroxide moiety), in particular the 6-membered heterocyclic ring, offers near optimal coupling between the unpaired electron spins. In addition, the trityl moiety offers much longer relaxation times as compared to the nitroxides which reduces the need for intense microwave irradiation when performing DNP experiments (G. Mathies et al., Angew. Chemie - Int. Ed., vol. 54, no. 40, pp. 11770-11774, 2015, doi: 10.1002/anie.201504292).

The chemical bridge or linker between Q₁ and Q₂ moieties present in the compounds of formula (I), in particular the 6-membered heterocyclic ring, provides stiffness and rigidity, which reduces the distance between the nitroxide unit Q₂ and the trityl unit Q₁ and promotes the efficient transfer of polarization. It is worth noting that the optimal polarizing agent structure is a complex multi-parameter problem, which includes the distance between the two radicals represented by Q₁ and Q₂, the intensity of the *J*-exchange interaction, the relative position and orientation of the g-tensors, as well as the associated electronic relaxation times and deuteration level of the polarizing agent.

With the aim to increase the interaction between unpaired electrons (called dipolar coupling and/or *J*-exchange interaction), the inventors found that in the compounds of formula (I), the number of atoms involved in the bridge or linker is reduced. As a result, the two electron spins are brought closer while keeping suitable g-tensor principal axis values to perform efficient CE DNP.

More importantly, in the compounds of formula (I), the bridge offers a significant advantage: these newly developed radicals can be readily synthesized and easily purified, in contrast to the known TEMTriPol-I described by G. Mathies et al., Angew. Chemie - Int. Ed., vol. 54, no. 40, pp. 11770-11774, 2015, doi: 10.1002/anie.201504292. The latter is not readily available and the synthesis includes a difficult and low-yielding HPLC-purification.

It is noteworthy that the newly developed radicals of the invention can be prepared as multiradicals, in which the trityl radical (Q₁) is connected to one, two and three nitroxides (Q₂), yielding bi-, tri- and tetraradicals, respectively, offering potentially improved DNP performance.

The compounds of formula (I) are suitable for any medium, i.e. water based or organic solvent based.

Thus, the new family of trityl-nitroxide(s) radicals provide readily accessible radicals for DNP at high magnetic field and fast MAS. These new radicals are particularly attractive when combined with low power microwave sources, such as solid-state microwave source.

Another object of the present invention is the use of a compound of formula (I) as a polarizing agent.

Another object of the invention is the use of a compound of formula (I) according to the invention as a polarizing agent (PA) for DNP in the context of structural biology, material sciences, Nuclear Magnetic Resonance of solids or applied to liquid samples, particle physics, and medical imaging.

In particular, the compounds of formula (I) may be used as DNP agents for polarizing an NMR-active isotope of a nucleus in Nuclear Magnetic Resonance (NMR) spectroscopy. The term NMR spectroscopy, as used herein, encompasses Solid State NMR (SS-NMR) spectroscopy, liquid state NMR spectroscopy and Magnetic Resonance Imaging (MRI), in all of which the compounds of the invention may be used as DNP agents.

A further object of the invention is a method for polarizing an analyte in a sample by Dynamic Nuclear Polarization comprising the steps of
a) providing a sample comprising an analyte;
b) contacting said sample with a compound of formula (I) as polarizing agent that enables an optimal nuclear polarization of the analyte in a magnetic field;
c) irradiating said sample with at least one radiation that causes electron spin flip, to enhance the performance of NMR detection or MRI performance; and
d) optionally dissolving the sample and obtaining a hyperpolarized sample.

### BRIEF DESCRIPTION OF THE DRAWINGS

The invention is further described by the following drawings and examples which illustrate embodiments thereof. These examples and drawings should not in any way be interpreted as limiting the scope of the present invention.
[Fig.1] represents the Electron Paramagnetic Resonance (EPR) spectrum in ClCH₂CH₂Cl, 1.0 mM, of piperazine nitroxide 2 prepared according the synthetic protocol described in example 1.
[Fig.2a] represents the EPR spectrum in ClCH₂CH₂Cl, 1.0 mM, of PyrroTriPol-OMe prepared according the synthetic protocol described in example 1.
[Fig.2b] represents the HPLC chromatogram of PyrroTriPol-OMe.
[Fig.3a] represents the EPR spectrum in H₂O, 1.0 mM, of PyrroTriPol prepared in example 1.
[Fig.3b] represents the HPLC chromatogram of PyrroTriPol.
[Fig.4] represents the EPR spectrum in ClCH₂CH₂Cl, 1.0 mM, of piperazine nitroxide 5 prepared according the synthetic protocol described in example 2.
[Fig.5a] represents the EPR spectrum in ClCH₂CH₂Cl, 1.0 mM, of PyrroTriPol-H-OMe prepared according the synthetic protocol described in example 2.
[Fig.5b] represents the HPLC chromatogram of PyrroTriPol-H-OMe.
[Fig.6a] represents the EPR spectrum in H₂O, 1.0 mM, of PyrroTriPol-H prepared in example 2.
[Fig.6b] represents the HPLC chromatogram of PyrroTriPol-H.
[Fig.7a] represents the EPR spectrum in ClCH₂CH₂Cl, 1.0 mM, of DiPyrroTriPol-OMe prepared according the synthetic protocol described in example 3.
[Fig.7b] represents the HPLC chromatogram of DiPyrroTriPol-OMe.
[Fig.8a] represents the EPR spectrum in ClCH₂CH₂Cl, 1.0 mM, of TriPyrroTriPol prepared according the synthetic protocol described in example 4.
[Fig.8b] represents the HPLC chromatogram of TriPyrroTriPol.
[Fig.9a] represents the EPR spectrum in ClCH₂CH₂Cl, 1.0 mM, of DiPyrroTriPol-H-OMe prepared according the synthetic protocol described in example 5.
[Fig.9b] represents the HPLC chromatogram of DiPyrroTriPol-H-OMe.
[Fig. 10a] represents the EPR spectrum in ClCH₂CH₂Cl, 1.0 mM, of TriPyrroTriPol-H prepared in example 6.
[Fig. 10b] represents the HPLC chromatogram of TriPyrroTriPol-H.

### DETAILED DESCRIPTION OF THE INVENTION

The present invention is a compound of formula (I) 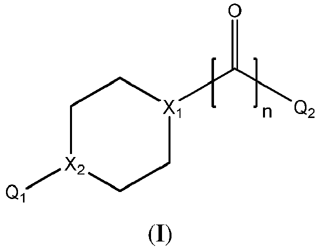 wherein
- n is 0 or 1;
- X₁ and X₂ are, independently, N, P, S;
- Q₂ is 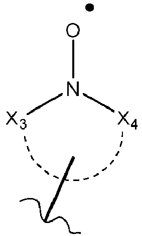 wherein
   - X₃ and X₄ are joined, as indicated by 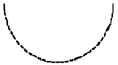 to form together with the nitrogen atom to which they are bound a 5- to 8-membered heterocyclic ring, the heterocyclic ring being optionally substituted, and/or
      the heterocyclic ring optionally containing one or more carbon-carbon double bond, 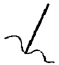
   - is the point of attachment of the 5- to 8-membered heterocyclic ring to the rest of the molecule;
- Q₁ is selected from 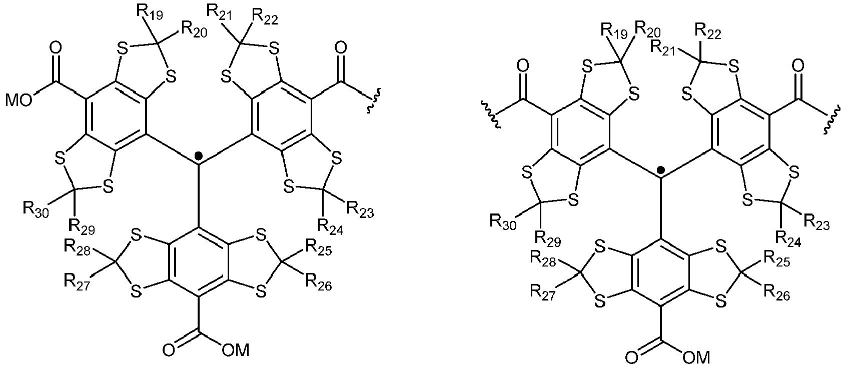 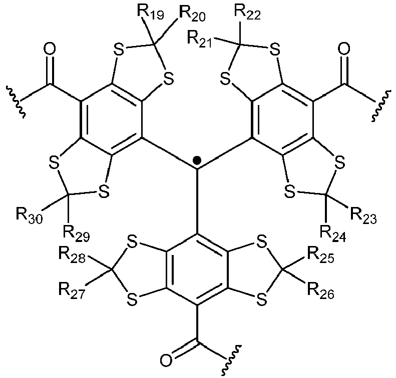 wherein
   - R₁₉ to R₃₀ are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms;
   - M is a hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms, an alkali cation selected from Na⁺ or K⁺, an ammonium cation (NH4)⁺;
   with the proviso that the compound of formula 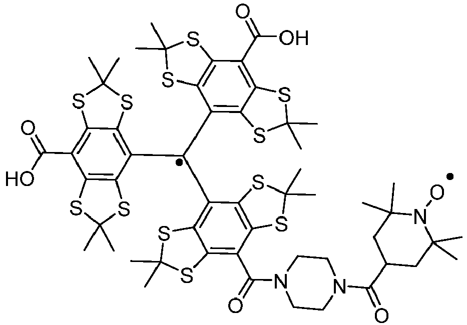 is excluded.

In the compounds of formula (I), the bridge/linker presented here as a saturated 6-membered ring containing heteroatoms in positions 1 and 4 (i.e. X₁ and X₂) provides stiffness and reduces flexibility by keeping an optimal distance between the trityl and nitroxide, and promotes the efficient transfer of polarization.

The compounds of formula (I) generate scalable synthesis of efficient trityl-nitroxides for aqueous and organic solvents with high *∈*_{on/off} amplification factors, minimized depolarization effects and fast nuclear polarization buildups.

As used herein, and unless otherwise indicated, the term "alkyl" means a saturated, linear, branched hydrocarbon having 1 to 10 carbon atoms, for example, 1 to 6 atoms. Examples of alkyls include, but are not limited to, methyl, ethyl, propyl, butyl, pentyl, hexyl, octyl, and their branched isomers such as isopropyl, 2-methyl-1-propyl, 2-methyl-2-propyl, 2-methyl-1-butyl, 3-methyl-1-butyl, 2-methyl-3-butyl, 2,2-dimethyl-1propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isobutyl, t-butyl, isopentyl, neopentyl.

As used herein, and unless otherwise indicated, the term "cycloalkyl" means a saturated cyclic hydrocarbon having 3 to 10 carbon atoms, for example, 3 to 6 carbon atoms. Examples of cyclic alkyls include, but are not limited to, cyclopropyl, cyclobutyl, cyclopentyl, cyclohexyl, cyclopropylmethyl.

An alkyl, may it be linear or branched, can be unsubstituted or substituted with one or more suitable substituents selected among halogen atoms such as fluorine, chlorine, bromine, iodine; hydroxyl; alkoxy; alkyl; cycloalkyl; alkylhalides; nitro (-NO₂); nitrile (-CN); aryl; CO-aryl; -CO-alkyl; -CO-cycloalkyl; -CO-alkoxy; -CO₂H; -CO₂Mₐ with Ma being an alkali metal selected in the group consisting of lithium (Li), sodium (Na), potassium (K); -NRaRbRc with Ra, R_{b} and R_{c} being, independently, an alkyl having 1 to 6 carbon atoms; -OPO₃(M_{b})₂ with M_{b} being a hydrogen, an alkali metal selected in the group consisting of lithium (Li), sodium (Na), potassium (K); with alkyl as defined above and hydroxyl, alkylhalides, alkoxy and aryl as defined hereinafter.

A cycloalkyl, can be unsubstituted or substituted with one or more suitable substituents selected among halogen atoms such as fluorine, chlorine, bromine, iodine; hydroxyl; alkoxy; alkyl; cycloalkyl; alkylhalides; nitro (-NO₂); nitrile (-CN); aryl; CO-aryl; -CO-alkyl; -CO-cycloalkyl; -CO-alkoxy; -CO₂H;-CO₂Mₐ with Ma being an alkali metal selected in the group consisting of lithium (Li), sodium (Na), potassium (K); -NRaRbRc with Ra, R_{b} and R_{c} being, independently, an alkyl having 1 to 6 carbon atoms; -OPO₃(M_{b})₂ with M_{b} being a hydrogen, an alkali metal selected in the group consisting of lithium (Li), sodium (Na), potassium (K); with alkyl as defined above and hydroxyl, alkylhalides, alkoxy and aryl as defined hereinafter.

As used herein, and unless otherwise indicated, the term "alkyl halide" refers to an alkyl or a "cycloalkyl" as described above, in which at least one hydrogen atom is substituted by a halogen atom selected from fluorine, chlorine, bromine and iodine. Non-limiting examples of alkyl halides are methyl fluoride, methyl chloride, methyl bromide, methyl iodide, ethyl fluoride, ethyl chloride, ethyl bromide, methyl difluoride, methyl dichloride, methyl chlorofluoride, methyl bromochlorofluoride, 2-chloropropyl, fluorocyclopentyl, (dibromomethyl)cyclohexyl, 2-iodo-2-methyl propyl, 2,4-dibromopentyl, methyl trifluoride, methyl trichloride, methyl tribromide, methyl triiodide.

As used herein, and unless otherwise indicated, the term "alkoxy" means an alkyl or a cycloalkyl as defined above, linked to another group via an oxygen atom (*i*.*e*. -O-(cyclo)alkyl).

As used herein, and unless otherwise indicated, the term "hydroxyl" means -OH. As used herein, and unless otherwise indicated, the term "halogen or halide" employed or in combination with other terms means fluorine, chlorine, bromine, iodine.

The term "heterocyclic ring", as used herein, refers to a non aromatic, partially unsaturated or fully saturated, 5-, 6-, 7- or 8- membered ring, for example 5- to 8-membered ring, or 5- to 6- membered ring wherein at least one ring atom is a heteroatom selected from oxygen, sulfur, and nitrogen. The remaining atoms of the heterocyclic ring are carbon atoms.

The heterocyclic ring may be partially unsaturated meaning that it may contain one or more unsaturated carbon-carbon bond. Preferably the unsaturated carbon-carbon bond is a carbon-carbon double bond (C=C). In some embodiments the heterocyclic ring contains one or more carbon-carbon double bonds. In some embodiments, the heterocyclic ring contains one carbon-carbon double bond.

In embodiments where the nitrogen atom present in the heterocyclic ring is a secondary nitrogen bearing a substituent Rₓ to form 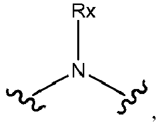 Rₓ can be hydrogen, hydroxyl, a substituted or unsubstituted linear, branched alkyl, a cycloalkyl as defined above, with 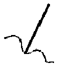 representing the points of attachment of nitrogen to the other members of the ring.

The heterocyclic ring is joined to the rest of the molecule via any of the carbon ring atoms.

Substituents on carbon atoms of the heterocyclic rings include alkyl; cycloalkyl; halogen atoms such as fluorine, chlorine, bromine, iodine; alkyl halide; alkoxy; hydroxyl; aryl; nitro (-NO₂); nitrile (-CN); -CO-aryl; -CO-alkyl; -CO-cycloalkyl; -CO-alkoxy; -CO₂H;-CO₂Mₐ with Ma being an alkali metal selected in the group consisting of lithium (Li), sodium (Na), potassium (K); -NRaRbRc with Ra, R_{b} and R_{c} being, independently, an alkyl having 1 to 6 carbon atoms; -OPO₃(M_{b})₂ with M_{b} being a hydrogen, an alkali metal selected in the group consisting of lithium (Li), sodium (Na), potassium (K); with hydroxyl, alkyl, cycloalkyl, alkyl halide, alkoxy, and aryl as defined herein.

As used herein, and unless otherwise indicated, the term "aryl" refers to an aromatic hydrocarbon having up to 14 carbon atoms, for example, 6 to 14 carbon atoms, which can be a single ring (monocyclic) or multiple rings (bicyclic, up to three rings) fused together or linked covalently. Any suitable ring position of the aryl moiety can be covalently linked to the defined chemical structure. Examples of aryl include, but are not limited, to phenyl, 1-naphtyl, 2-naphtyl, dihydronaphtyl, tetrahydronaphtyl, biphenyl, anthryl, phenanthryl. An aryl can be unsubstituted substituted with one or more suitable substituents including halogen atoms such as fluorine, chlorine, bromine, iodine; hydroxyl; alkyl; cycloalkyl; alkyl halide; alkoxy; aryl; nitro (-NO₂); nitrile (-CN); -CO-aryl; -CO-alkyl; -CO-cycloalkyl; -CO-alkoxy; -CO₂H;-CO₂Mₐ with Ma being an alkali metal selected in the group consisting of lithium (Li), sodium (Na), potassium (K); -NRaRbRc with Rₐ, R_{b} and R_{c} being independently an alkyl having 1 to 6 carbon atoms; -OPO₃(M_{b})₂ with M_{b} being a hydrogen, an alkali metal selected in the group consisting of lithium (Li), sodium (Na), potassium (K); with hydroxyl, alkyl, cycloalkyl, alkyl halide, alkoxy, and aryl as defined herein. Non-limiting examples of substituted aryl can be tolyl, methoxyphenyl, dimethoxy phenyl, trimethoxyphenyl, fluorophenyl, difluorophenyl, methyltrifluoride phenyl, nitrophenyl, methylnitrophenyl, methoxynitrophenyl, dimethoxynitrophenyl chloronitrophenyl, nitrilphenyl, tolylnitrophenyl, methoxynapthtyl, -CO-phenyl.

In a **first** embodiment of the invention, in the compound of formula (I), Q₂ is selected from 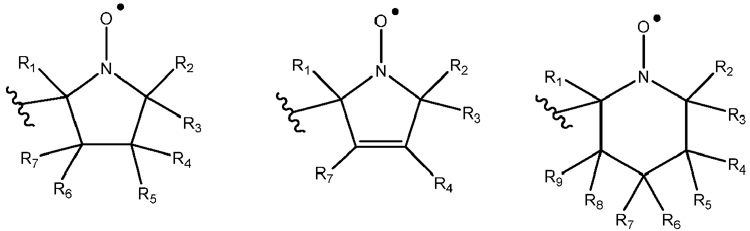 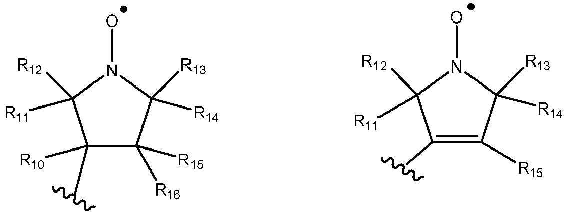 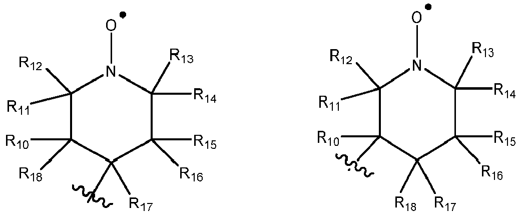 wherein
- R₁ to R₁₈ are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms; or
- R₁ to R₉, R₁₀, and R₁₅ to R₁₈, are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms, and
   geminal R₁₁ and R₁₂, and geminal R₁₃ and R₁₄, are joined to form together with the secondary carbon to which they are bound a substituted or unsubstituted cycloalkyl having 3 to 10 carbon atoms, a substituted or unsubstituted 5- or 6-membered heterocyclic ring wherein at least one ring atom is oxygen. 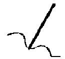 is the point of attachment of the 5- to 6-membered heterocyclic ring to the rest of the molecule.

In this first embodiment, n, X₁, X₂ and Q₁ are as described above.

In a **second** embodiment of the invention, in the compound of formula (I), Q₂ is selected from 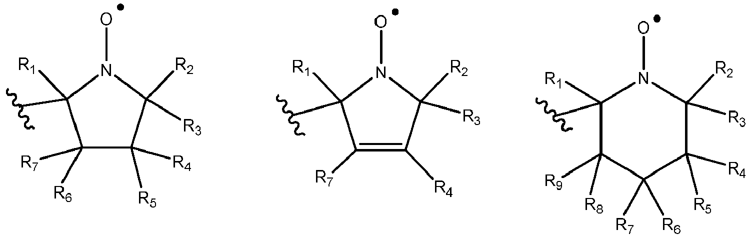 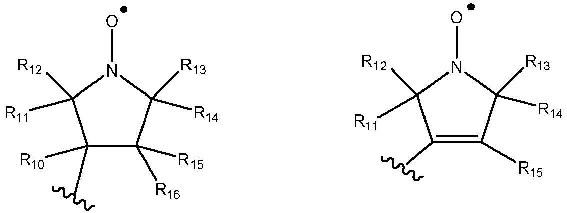 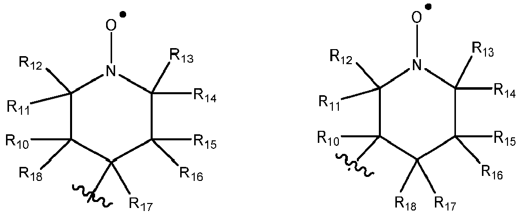 wherein R₁ to R₁₈ are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms. 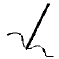 is the point of attachment of the 5- to 6-membered heterocyclic ring to the rest of the molecule.

In this second embodiment, n, X₁, X₂ and Q₁ are as described above.

Preferably, R₁ to R₁₈ are, independently, hydrogen, a substituted or unsubstituted linear, branched alkyl having 1 to 6 carbon atoms, a substituted or unsubstituted cycloalkyl having 3 to 6 carbon atoms, a substituted or unsubstituted aryl having 6 to 14 carbon atoms.

More preferably, R₁ to R₁₈ are, independently, hydrogen, a substituted or unsubstituted linear, branched alkyl having 1 to 6 carbon atoms.

In a **third** embodiment of the invention, Q₂ is selected from 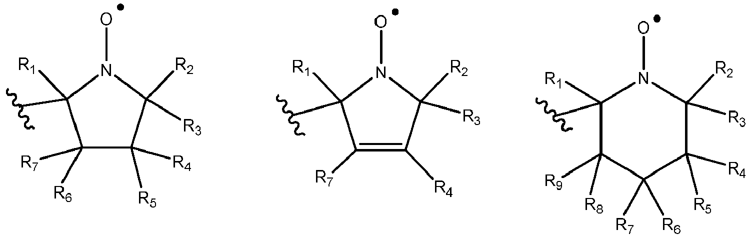 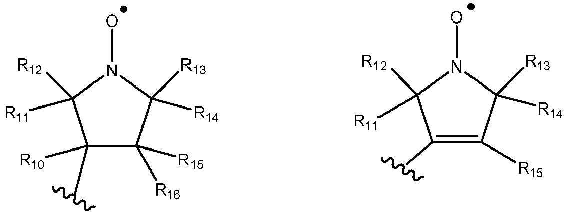 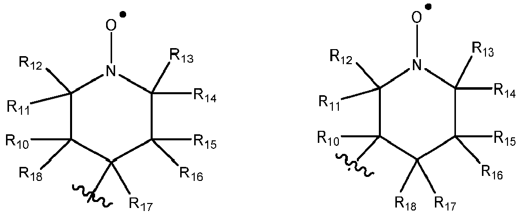 wherein
- R₁ to R₉, R₁₀, and R₁₅ to R₁₈, are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms, and
   geminal R₁₁ and R₁₂, and geminal R₁₃ and R₁₄, are joined to form together with the secondary carbon to which they are bound a substituted or unsubstituted cycloalkyl having 3 to 10 carbon atoms, a substituted or unsubstituted 5- or 6-membered heterocyclic ring wherein at least one ring atom is oxygen.

In this third embodiment, n, X₁, X₂ and Q₁ are as described above. 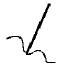 is the point of attachment of the 5- to 6-membered heterocyclic ring to the rest of the molecule.

Preferably,
- R₁ to R₉, R₁₀, and R₁₅ to R₁₈, are, independently, hydrogen, a substituted or unsubstituted linear, branched alkyl having 1 to 6 carbon atoms, a substituted or unsubstituted cycloalkyl having 3 to 6 carbon atoms, a substituted or unsubstituted aryl having 6 to 14 carbon atoms, and
   geminal R₁₁ and R₁₂, and geminal R₁₃ and R₁₄, are joined to form together with the secondary carbon to which they are bound a substituted or unsubstituted cycloalkyl having 3 to 6 carbon atoms, a substituted or unsubstituted 5- or 6-membered heterocyclic ring wherein at least one ring atom is oxygen.

More preferably, R₁ to R₉, R₁₀, and R₁₅ to R₁₈, are, independently, hydrogen, a substituted or unsubstituted linear, branched alkyl having 1 to 6 carbon atoms, and geminal R₁₁ and R₁₂, and geminal R₁₃ and R₁₄, are joined to form together with the secondary carbon to which they are bound, a substituted or unsubstituted cycloalkyl having 3 to 6 carbon atoms, a substituted or unsubstituted 5- or 6-membered heterocyclic ring with one ring atom being oxygen.

When geminal R₁₁ and R₁₂, and geminal R₁₃ and R₁₄, are joined to form together with the secondary carbon to which they are bound a substituted cycloalkyl having 3 to 6 carbon atoms, a substituted 5- or 6-membered heterocyclic ring with one ring atom being oxygen, said cycloalkyl and said heterocyclic ring are preferably substituted with one or more suitable substituents including
- halogen atoms such as fluorine, chlorine, bromine, iodine;
- hydroxyl;
- unsubstituted alkyl having 1 to 6 carbon atoms;
- substituted alkyl having 1 to 6 carbon atoms, the substitution including CO₂Mₐ with Ma being an alkali metal selected in the group consisting of lithium (Li), sodium (Na), potassium (K); -NRaRbRc with Ra, R_{b} and R_{c} being, independently, an alkyl having 1 to 6 carbon atoms; -OPO₃(M_{b})₂ with M_{b} being a hydrogen, an alkali metal selected in the group consisting of lithium (Li), sodium (Na), potassium (K);
- cycloalkyl;
- alkyl halide;
- alkoxy;
- aryl;
- nitro (-NO₂);
- nitrile (-CN);
- -CO-aryl;
- -CO-alkyl;
- -CO-cycloalkyl;
- -CO-alkoxy;
- -CO₂H;
- -CO₂Mₐ with Ma being an alkali metal selected in the group consisting of lithium (Li), sodium (Na), potassium (K);
- -NRaRbRc with Ra, R_{b} and R_{c} being independently an alkyl having 1 to 6 carbon atoms;
- -OPO₃(M_{b})₂ with Mb being a hydrogen, an alkali metal selected in the group consisting of lithium (Li), sodium (Na), potassium (K);
with hydroxyl, alkyl, cycloalkyl, alkyl halide, alkoxy, and aryl as defined herein.

In a **fourth** embodiment of the invention, Q₂ is selected from 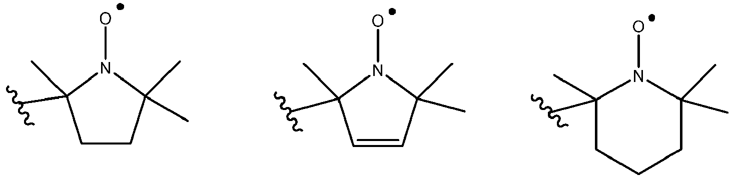 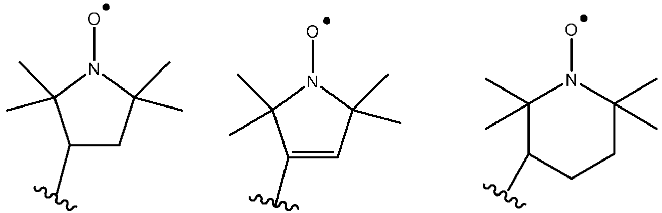 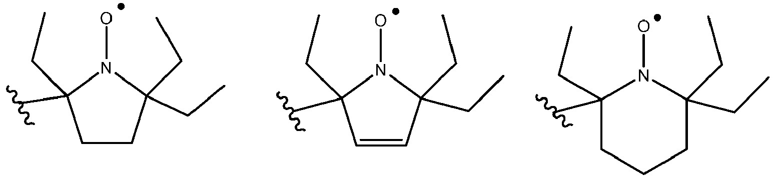 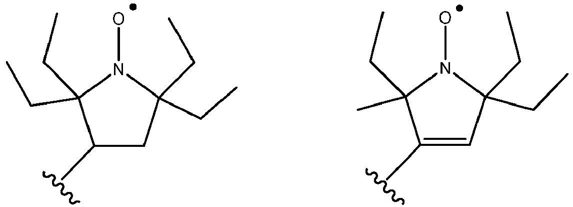 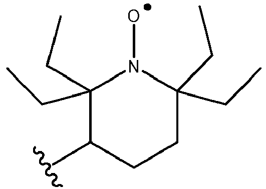 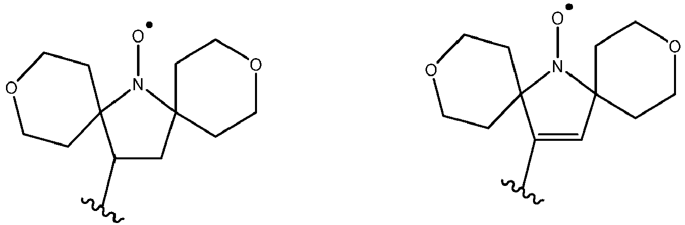 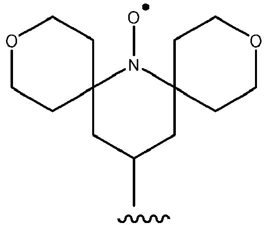 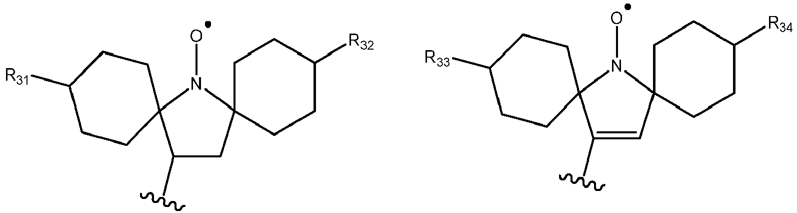 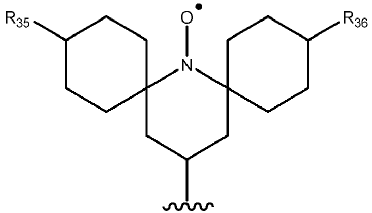 with R₃₁ to R₃₆, independently, representing
- a hydrogen;
- a hydroxyl;
- an alkoxy with the alkyl being , methyl, ethyl, propyl, butyl, pentyl, hexyl, and their branched isomers;
- an unsubstituted alkyl selected from methyl, ethyl, propyl, butyl, pentyl, hexyl, and their branched isomers;
- a substituted alkyl selected from methyl, ethyl, propyl, butyl, pentyl, hexyl, and their branched isomers, the substitution including CO₂Mₐ with Ma being an alkali metal selected in the group consisting of sodium (Na), potassium (K); -NRaRbRc with Rₐ, R_{b} and R_{c} being, independently, methyl, ethyl, propyl, butyl, pentyl, hexyl, and their branched isomers; -OPO₃(M_{b})₂ with Mb being a hydrogen, an alkali metal selected in the group consisting of sodium (Na), potassium (K);
- CO₂Mₐ with Ma being an alkali metal selected in the group consisting of sodium (Na), potassium (K);
- -NRaRbRc with Rₐ, R_{b} and R_{c} being, independently, methyl, ethyl, propyl, butyl, pentyl, hexyl, and their branched isomers;
- -OPO₃(M_{b})₂ with Mb being a hydrogen, an alkali metal selected in the group consisting of sodium (Na), potassium (K).

In this fourth embodiment, n, X₁, X₂ and Q₁ are as described above. 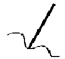 is the point of attachment of the 5- to 6-membered heterocyclic ring to the rest of the molecule.

In a **fifth** embodiment of the invention, Q₁ is selected from 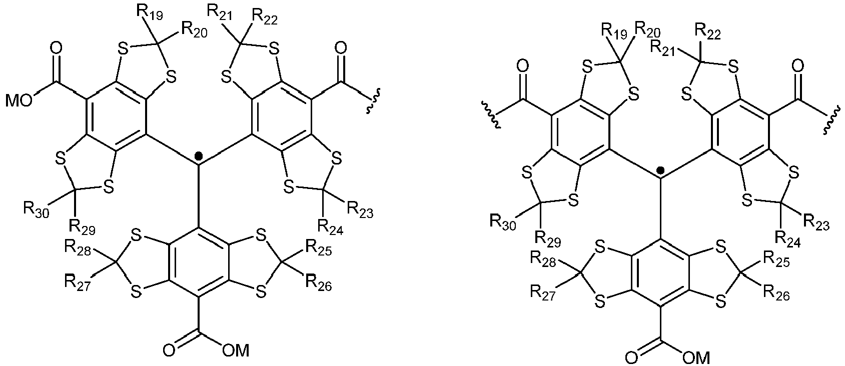 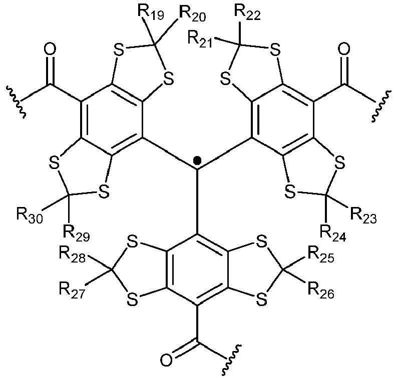 wherein
- R₁₉ to R₃₀ are, independently, hydrogen, a substituted or unsubstituted linear, branched alkyl having 1 to 6 carbon atoms, a substituted or unsubstituted cycloalkyl having 3 to 6 carbon atoms, a substituted or unsubstituted aryl having 6 to 14 carbon atoms;
- M is a hydrogen, a substituted or unsubstituted linear, branched alkyl having 1 to 6 carbon atoms, a substituted or unsubstituted cycloalkyl having 3 to 6 carbon atoms, a substituted or unsubstituted aryl having 6 to 14 carbon atoms, an alkali cation selected from Na⁺ or K⁺, an ammonium cation (NH₄)⁺.

In this fifth embodiment, n, X₁, X₂ and Q₂ are as described above. 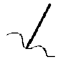 is the point of attachment of the ―(C=O) group to the rest of the molecule.

In a **sixth** embodiment of the invention, Q₁ is selected from 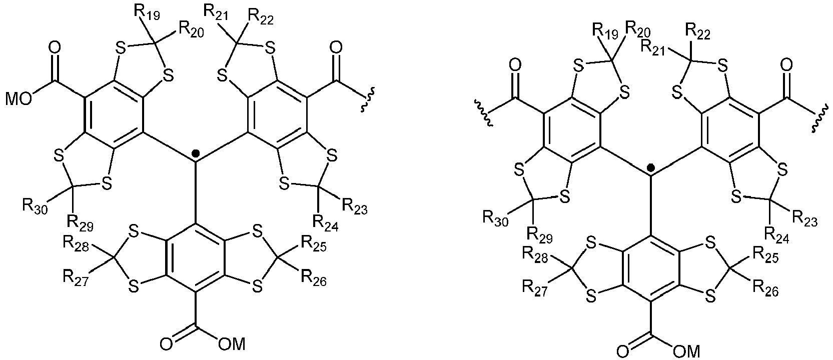 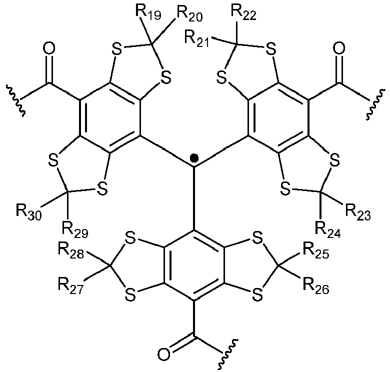 wherein
- R₁₉ to R₃₀ are, independently, hydrogen, a substituted or unsubstituted linear, branched alkyl having 1 to 6 carbon atoms;
- M is a hydrogen, a substituted or unsubstituted linear, branched alkyl having 1 to 6 carbon atoms, an alkali cation selected from Na⁺ or K⁺, an ammonium cation (NH₄)⁺.

In this sixth embodiment, n, X₁, X₂ and Q₂ are as described above. 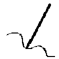 is the point of attachment of the ―(C=O) group(s) to the rest of the molecule.

In a **seventh** embodiment of the invention, Q₁ is selected from 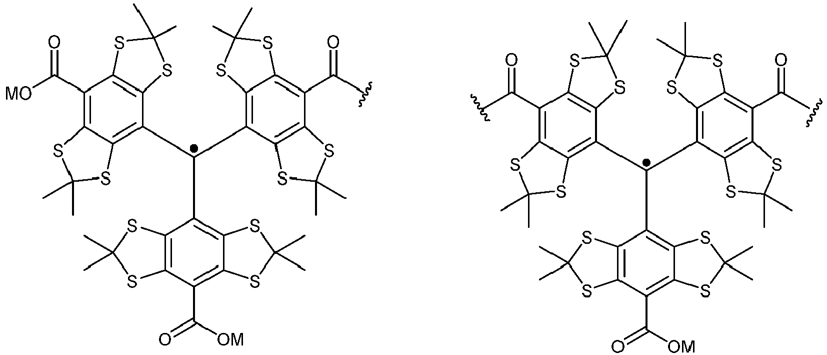 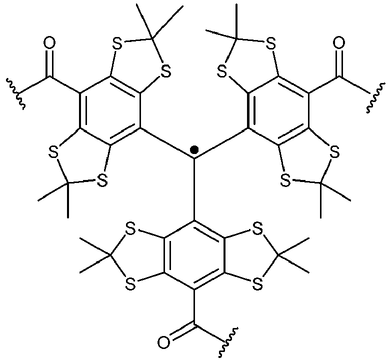 wherein M is a hydrogen, an unsubstituted alkyl selected from methyl, ethyl, propyl, butyl, pentyl, hexyl, and their branched isomers, an alkali cation selected from Na⁺ or K⁺, an ammonium cation (NH₄)⁺.

In this seventh embodiment, n, X₁, X₂ and Q₂ are as described above. 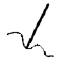 is the point of attachment of the ―(C=O) group(s) to the rest of the molecule.

In all the embodiments disclosed herein, X₁ and X₂ are preferably N. As already indicated, the compound of formula 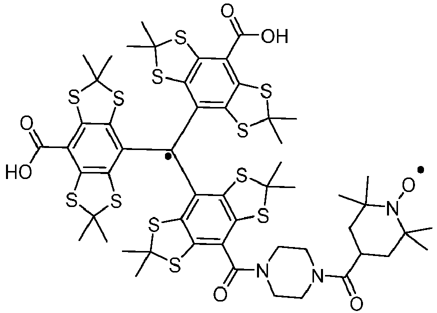 is not part of the invention and is excluded from all of the embodiments disclosed herein.

In an exemplary embodiment according to the invention, the compound of formula (I) is

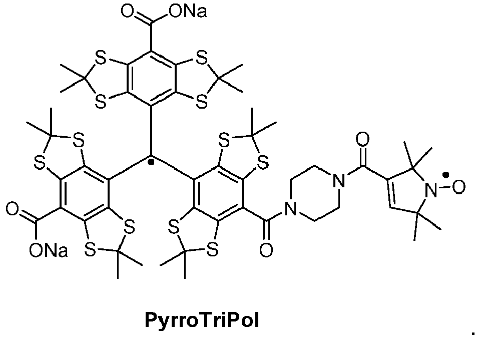

In an exemplary embodiment according to the invention, the compound of formula (I) is

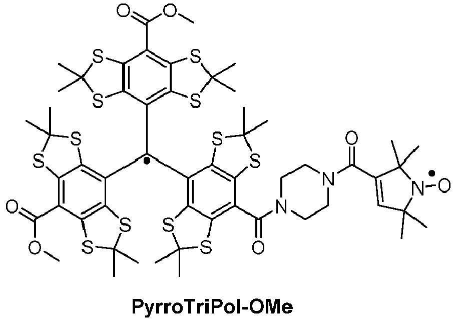

In an exemplary embodiment according to the invention, the compound of formula (I) is

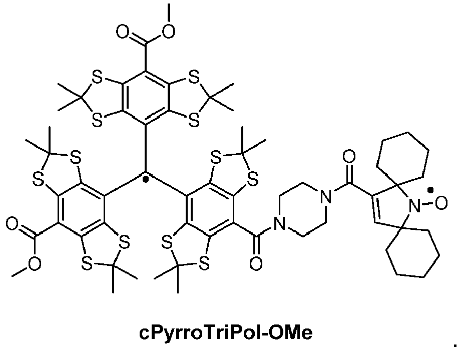

In an exemplary embodiment according to the invention, the compound of formula (I) is

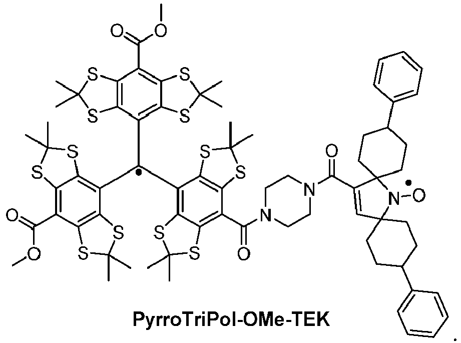

In an exemplary embodiment according to the invention, the compound of formula (I) is

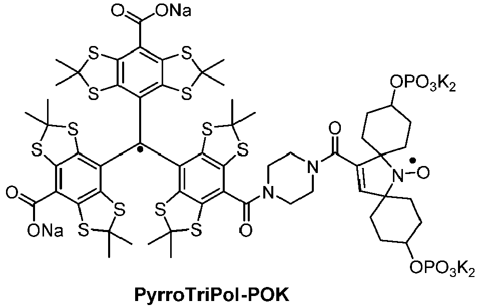

In an exemplary embodiment according to the invention, the compound of formula (I) is

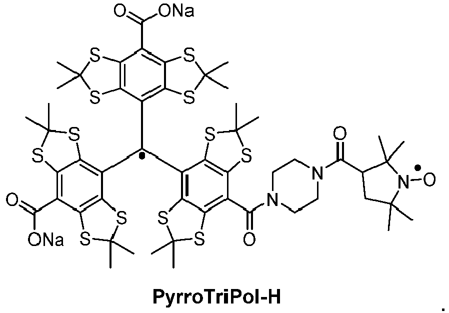

In an exemplary embodiment according to the invention, the compound of formula (I) is

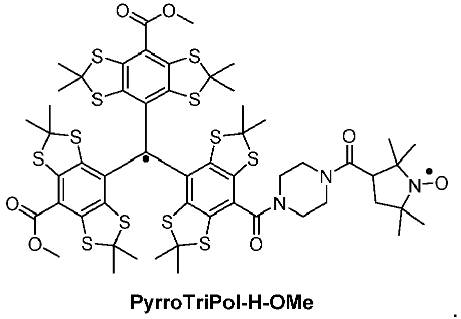

In an exemplary embodiment according to the invention, the compound of formula (I) is

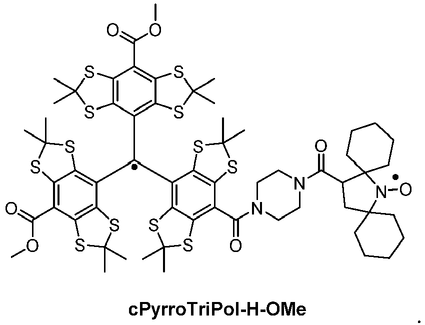

In an exemplary embodiment according to the invention, the compound of formula (I) is

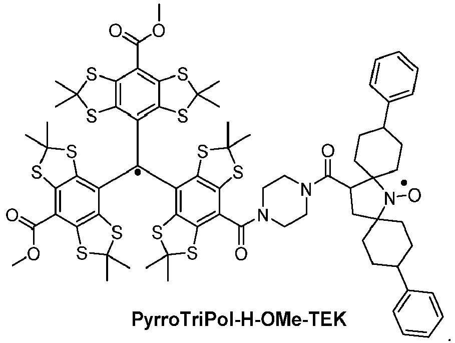

In an exemplary embodiment according to the invention, the compound of formula (I) is

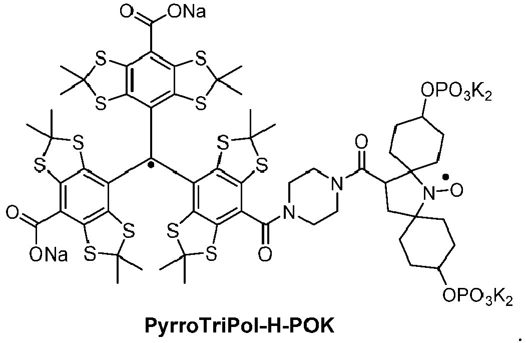

In an exemplary embodiment according to the invention, the compound of formula (I) is

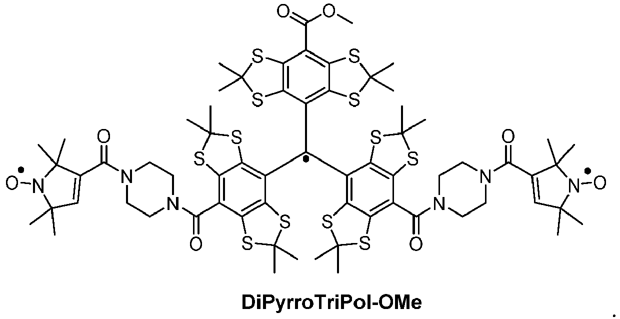

In an exemplary embodiment according to the invention, the compound of formula (I) is

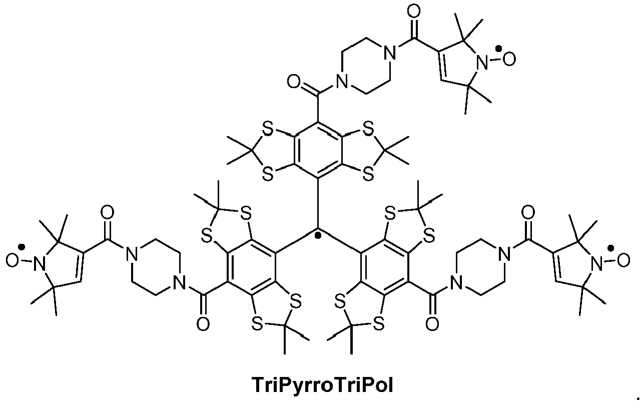

In an exemplary embodiment according to the invention, the compound of formula (I) is

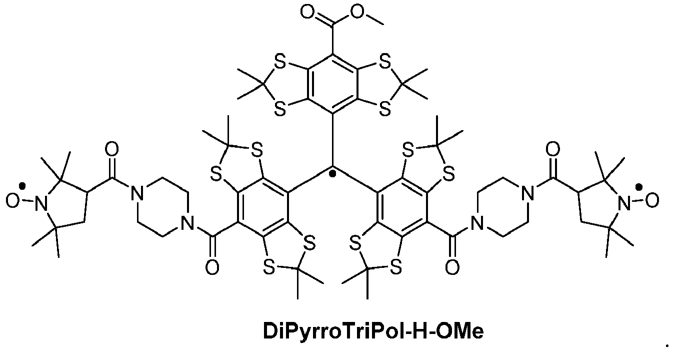

In an exemplary embodiment according to the invention, the compound of formula (I) is

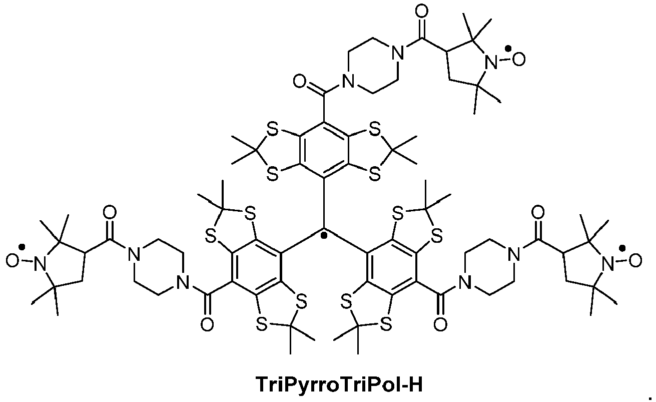

The nitroxide-carbon-based biradicals proposed up to now offer low to moderate performance at high field and fast MAS. More importantly, their widespread use has been so far limited since they suffer either from limited chemical stability (HyTEK) or they require a tedious synthesis. Both aspects have prevented the commercialization of these radicals so far.

The compounds of formula (I) address both problems of limited efficiency and stability of nitroxide-carbon-based biradicals. In addition, the synthetic route for these compounds is compatible with large scale synthesis and thus commercialization.

Syntheses of particular compounds of formula (I) according to the invention are described in details in the examples.

A general method for the synthesis of the compounds of the invention can be obtained according to a one-step synthesis as described below.

The solvents used for the reaction may be the same or selected from diethylether, dimethylether, dioxane, dichloromethane, dichloroethane, acetonitrile, chloroform, dimethylformamide (DMF), dimethylsulfoxide (DMSO), tetrahydrofurane (THF) or toluene.

The reaction temperature can be between 20 and 50 °C, preferably between 20 and 30 °C.

The reaction time can be between 1 and 48 h, more preferably between 6 and 24 h. The molar ratio between the trityl compound and the nitroxide radical can be between 1 and 6, in particular between 1 and 1.2.

The compounds of formula (I) can be isolated and purified, if necessary, by conventional separation/purification methods used in the synthetic organic chemistry, such as filtration, extraction, washing, drying, concentration, recrystallization, various chromatographic techniques or the like.

The compounds of the invention are stable and are soluble either in aqueous or organic solvent.

Another object of the present invention relates to the use of a compound of formula (I) as a polarizing agent.

Another object of the invention is the use of a compound of formula (I) according to the invention as a PA for DNP in the context of structural biology, material sciences, Nuclear Magnetic Resonance of solids or applied to liquid samples, particle physics, and medical imaging. In particular, the compounds of formula (I) may be used as DNP agents for polarizing an NMR-active isotope of a nucleus in Nuclear Magnetic Resonance (NMR) spectroscopy. The term NMR spectroscopy, as used herein, encompasses Solid State NMR (SS-NMR) spectroscopy, liquid state NMR spectroscopy and Magnetic Resonance Imaging (MRI), in all of which the compounds of the invention may be used as DNP agents.

A nucleus having an NMR-active spin may be, for example: ¹H, ²H, ⁶Li, ⁷Li, ¹⁰B, ¹¹B_{,} ¹³C, ¹⁴N, ¹⁵N, ¹⁷O, ¹⁹F, ²³Na, ²⁵Mg, ²⁷Al, ²⁹Si, ³¹P, ³³S, ³⁵Cl, ³⁷Cl, ³⁹K, ⁴¹K, ⁴³Ca, ⁴⁷Ti, ⁴⁹Ti, ⁵⁰V, ⁵¹V, ⁵³Cr, ⁷⁷Se, ⁸⁹Y, ¹¹⁷Sn, ¹¹⁹Sn and ¹⁹⁹Hg.

A further object of the invention relates to a method for polarizing an analyte in a sample for Dynamic Nuclear Polarization comprising the steps of
a) providing a sample comprising an analyte;
b) contacting said sample with a compound of formula (I) as polarizing agent that enables an optimal nuclear polarization of the analyte in a magnetic field;
c) irradiating said sample with at least one radiation that causes electron spin flip, to enhance the performance of NMR detection or MRI performance; and
d) optionally dissolving the sample and obtaining a hyperpolarized sample.

Said method optionally further comprises observing the NMR or MRI of the hyperpolarized sample.

The irradiation is preferably a microwave irradiation. The frequency range of the microwave irradiation by which the polarization is transferred to an NMR-active nucleus is usually from 5 to 800 GHz.

The term "analyte", as used herein, refers to a chemical or a biological entity, such as a solid inorganic, organic or metallo-organic material having a crystal lattice or an amorphous solid structure (e.g. zeolites, nanoparticles, mesoporous and porous materials, glasses, Metal Organic Frameworks (MOF), a molecular chemical or biochemical compound including polymeric compounds and macromolecular compounds (e.g. proteins, enzymes, DNA/RNA and a biological entity (e.g. a whole cell, a leaf, a virus particle, tissue or bone components or a whole body, having one or more NMR-active spins to be investigated by NMR spectroscopy)). The chemical or a biological entity may be isolated or in its natural environment. The analyte may be dissolved in aqueous medium, an organic solvent or solvent mixture or an aqueous/organic solvent mixture. The analyte may be present in the sample without a solvent.

The investigation by NMR spectroscopy may be structure determination, monitoring of reaction kinetics, flow imaging, etc.

The polarizing agent may be dissolved in the solvent(s) of the sample or may be introduced without a solvent chemically bound to the analyte, such as in doped polymers, materials functionalized with polarizing agents, or paramagnetic spin labels on biological samples.

The polarizing agent may also be dispersed in the analyte, for example by initially introducing the polarizing agent with a solvent and evaporating the solvent in a following step leaving the polarizing agent and analyte, or be introduced by wet impregnation. The polarizing agent may also be added during a synthetic preparation step.

The polarizing agent may be present in a solid state during the polarization time, such as in a frozen solution comprising a frozen solvent or solvent mixture containing the analyte or in a solid state.

The polarizing agent may be present in a liquid state or in a liquid solution during the polarization time.

A compound of formula (I) according to the invention, when used as a polarizing agent, is used at a concentration of 0.01 to 200 mM.

In solid state NMR experiments, the temperature of a sample including the polarizing agent is in the range of 1 to 300 K.

The invention will be further illustrated by the following figures and examples.

### EXAMPLES

All commercially available reagents were purchased from Sigma-Aldrich, Inc. or Acros Organics and used without further purification. All moisture- and air-sensitive reactions were carried out in oven-dried glassware under an inert atmosphere of Ar. Thin-layer chromatography (TLC) was performed using glass plates pre-coated with silica gel (0.25 mm, F-25, Silicycle) and compounds were visualized under UV light. Column chromatography was performed using 230-400 mesh silica gel (Silicycle). Radicals show broadening and loss of NMR signals due to their paramagnetic nature and, therefore, those NMR spectra are not shown. EPR spectra were recorded on a MiniScope MS200 (Magnettech Germany) spectrometer. Mass spectrometric analyses of all organic compounds were performed on an ESI-HRMS (Bruker, MicrOTOF-Q) in a positive or negative ion mode.

Purification of **PyrroTriPol** and **PyrroTriPol-H** was performed on a preparative Agilent HPLC system using a GL Sciences Inertsustain C18 14×250 mm column with UV detection at λ = 254 nm with a flow rate of 10 mL/min using the following gradient: Solvent A, H₂O; solvent B, CH₃CN; 0-4 min isocratic 4% B, 4-20 min gradient 4-100% B, 20-21 min isocratic 100% B, 21-23 min 100-4% B. Purity of all biradicals was analyzed on an analytical Agilent HPLC system using a Pursuit 5 C18 4.6x250 mm analytical column with UV detection at λ = 254 nm with a flow rate of 1 mL/min using an isocratic run for **PyrroTriPol-OMe, DiPyrroTriPol-OMe, TriPyrroTriPol, PyrroTriPol-H-OMe, DiPyrroTriPol-H-OMe** and **TriPyrroTriPol-H:** 0-16 min 100%.

### Example 1:

### Synthesis of piperazine nitroxide 2

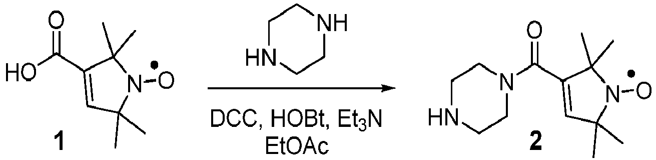

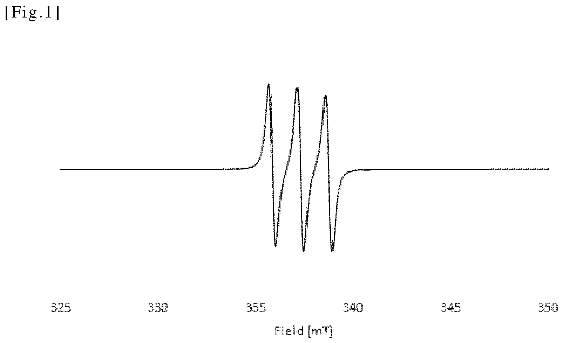

To a solution of nitroxide **1** (doi: org/10.1016/j.saa.2008.07.045) (0.1 00 g, 0.54 mmol) in EtOAc (5.0 mL) was added *N,N'*-dicyclohexylcarbodiimide or DCC (0.167 g, 0.81 mmol) and *N*-hydroxybenzotriazole or HOBt (0.124 g, 0.81 mmol) and the resulting solution was stirred at 22 °C for 5 min. and added dropwise to a solution of piperazine (0.140 g, 1.63 mmol) in EtOAc (10 mL) and Et3N (0.11 mL, 0.81 mmol). The reaction mixture was stirred at 22 °C for 12 h. The precipitate was filtered off, the solvent was removed *in vacuo* and the residue purified by column chromatography (CH₂Cl₂:MeOH, 9:1 + 0.1% Et3N) to yield piperazine nitroxide **2** (0.108 g, 0.43 mmol, 79%) as yellow solid.
TLC (Silica gel, CH₂Cl₂: MeOH 9:1), Rf (**piperazine nitroxide 2**) = 0.1 ESI-HRMS: calcd. for C₁₃H₂₂N₃O₂ [M+H⁺] 253.1785, measured 253.1776 (Δm = 0.0009, error = 3.6 ppm).
EPR (ClCH₂CH₂Cl, 1.0 mM): EPR spectrum represented in [Fig.1].

### Synthesis of PyrroTriPol-OMe

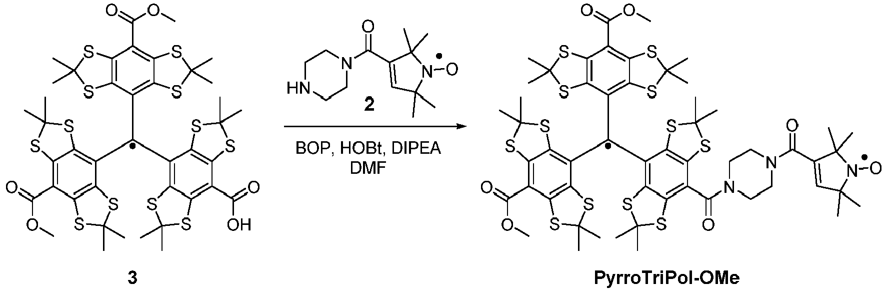

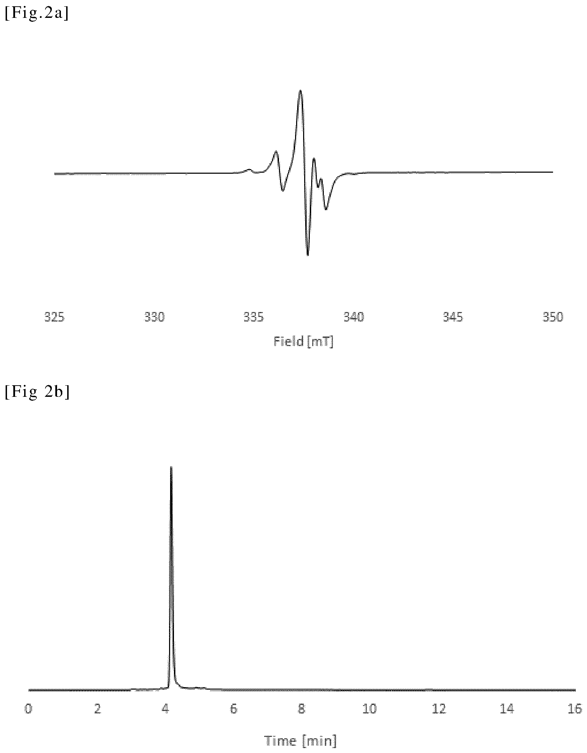

To a solution of trityl **3** (doi: 10.1055/s-0035-1561299) (0.030 g, 0.029 mmol) in DMF (2.0 mL) was added benzotriazol-1-yloxytris(dimethylamino)phosphonium hexafluorophosphate (0.020 g, 0.044 mmol), N-hydroxybenzotriazole (0.006 g, 0.044 mmol) and DIPEA (0.008 mL, 0.044 mmol) and the resulting solution was stirred at 22 °C for 5 min. Nitroxide **2** (0.008 g, 0.032 mmol) was added to the solution and the resulting reaction mixture was stirred at 22°C for 12 h. Saturated aqueous NaHCO₃ (10 mL) was added and the solution extracted with EtOAc (3×10 mL). The combined organic layers were dried over Na₂SO₄, the solvent was removed *in vacuo* and the residue purified by flash column chromatography (petroleum ether: EtOAc, 6:4) to give **PyrroTriPol-OMe** (0.033 g,0.026 mmol, 88% yield) as a green solid.
TLC (Silica gel, pet. ether : EtOAc 1 : 1), Rf (**PyrroTriPol-OMe**) = 0.4 ESI-HRMS: calcd. for C₅₅H₆₃N₃O₇S₁₂ [M+Na⁺] 1284.1207, measured 1284.1164 (Δm = 0.0043, error = 3.3 ppm).
EPR (ClCH₂CH₂Cl, 1.0 mM): EPR spectrum represented in [Fig.2a].
HPLC: HPLC chromatogram represented in [Fig.2b].

### Synthesis of PyrroTriPol

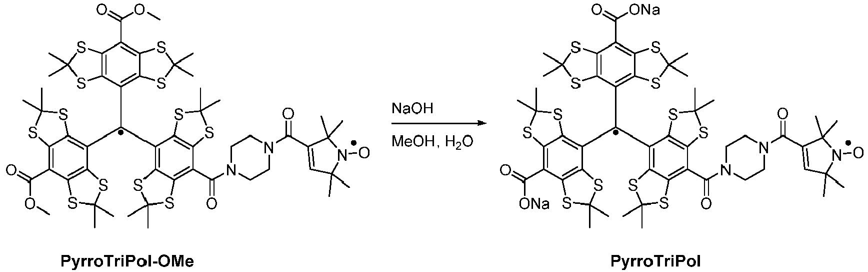

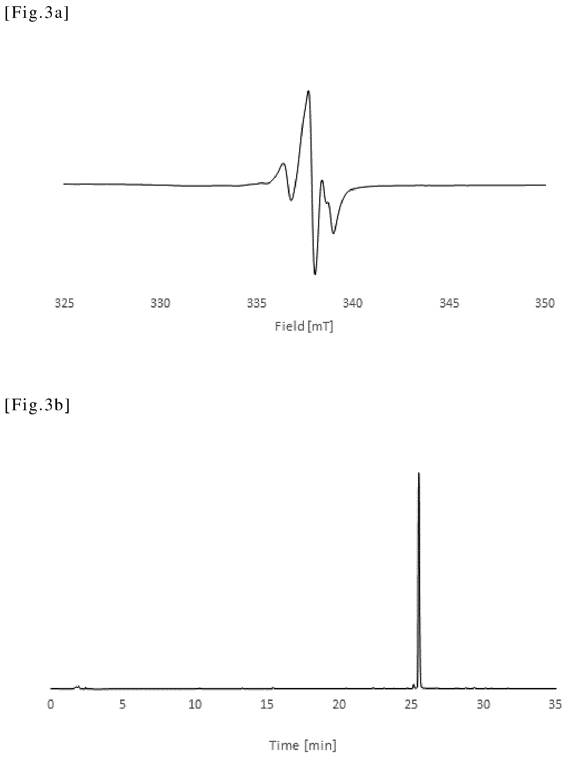

To a solution of **PyrroTriPol-OMe** (0.015 g, 0.012 mmol) in MeOH (2.0 mL) was added NaOH (0.003 g, 0.072 mmol) and H₂O (0.1 mL). The reaction mixture was stirred at 22 °C for 48 h. The solvent was removed *in vacuo* and the residue purified by C18-HPLC to afford **PyrroTriPol** (0.013 g, 0.010 mmol, 86% yield) as a green solid.
ESI-HRMS: calcd. for C₅₃H₅₉N₃O₇S₁₂ [M-H⁺] 1232.0929, measured 1232.0922 (Δm = 0.0007, error = 0.6 ppm).
EPR (H₂O, 1.0 mM): EPR spectrum represented in [Fig.3a].
HPLC: HPLC chromatogram represented in [Fig.3b].

### Example 2:

### Synthesis of piperazine nitroxide 5

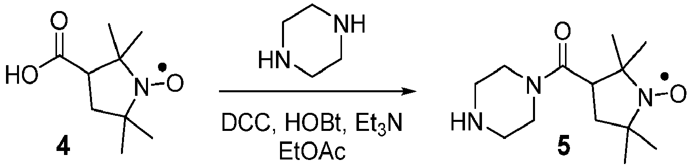

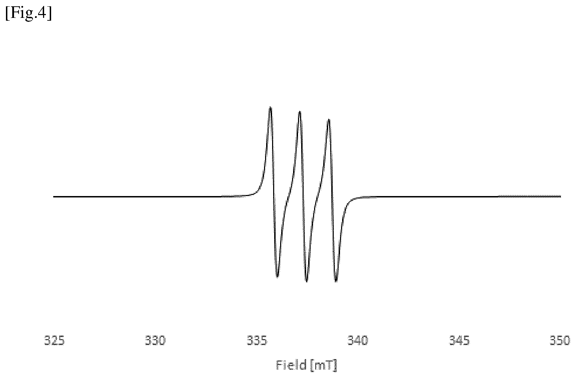

To a solution of nitroxide **4** (doi:org/10.1039/C7CP07444A) (0.100 g, 0.54 mmol) in EtOAc (5.0 mL) was added *N,N'*-dicyclohexylcarbodiimide or DCC (0.167 g, 0.81 mmol) and *N*-hydroxybenzotriazole or HOBt (0.124 g, 0.81 mmol) and the mixture stirred at 22 °C for 5 min. and added dropwise to a solution of piperazine (0.140 g, 1.63 mmol) in EtOAc (10 mL) and Et3N (0.11 mL, 0.81 mmol). The resulting mixture was stirred at 22 °C for 12 h. The precipitate was filtered off, the solvent was removed *in vacuo* and purified by column chromatography (CH₂Cl₂:MeOH, 9:1 + 0.1% Et3N) to yield piperazine nitroxide **2** (0.099 g, 0.39 mmol, 72%) as yellow solid.
TLC (Silica gel, CH₂Cl₂:MeOH 9:1), Rf (**piperazine nitroxide 5**) = 0.1 ESI-HRMS: calcd. for C₁₃H₂₄N₃O₂ [M+Na⁺] 277.1761, measured 277.1760 (Δm = 0.0001, error = 0.4 ppm).
EPR (ClCH₂CH₂Cl, 1.0 mM): EPR spectrum represented in [Fig.4].

### Synthesis of PyrroTriPol-H-OMe

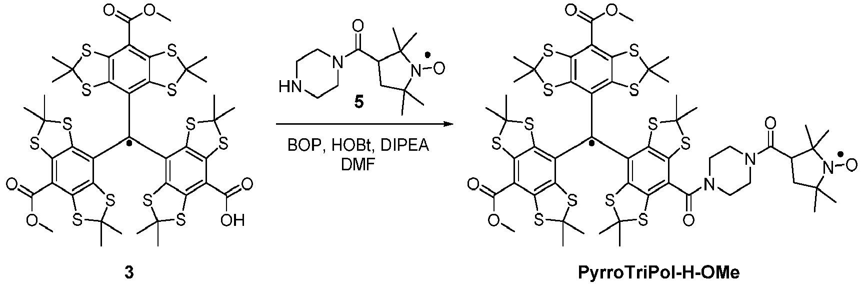

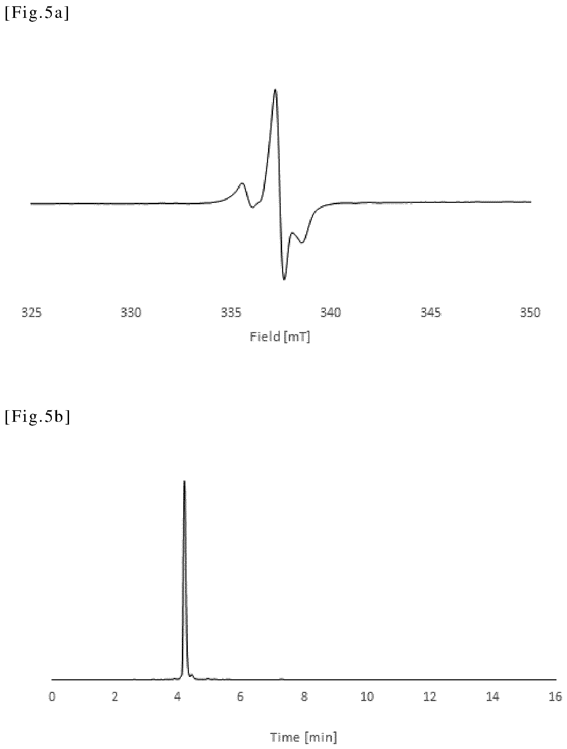

To a solution of trityl **3** (doi: 10.1055/s-0035-1561299) (0.030 g, 0.029 mmol) in DMF (2.0 mL) was added benzotriazol-1-yloxytris(dimethylamino)phosphonium hexafluorophosphate (0.020 g, 0.044 mmol), *N*-hydroxybenzotriazole (0.006 g, 0.044 mmol) and DIPEA (0.008 mL, 0.044 mmol) and the resulting solution stirred at 22 °C for 5 min. Nitroxide **5** (0.008 g, 0.032 mmol) was added to the solution and the resulting reaction mixture was stirred at 22 °C for 12 h. Saturated aqueous NaHCO₃ (10 mL) was added and the solution extracted with EtOAc (3×10 mL). The combined organic layers were dried over Na₂SO₄, the solvent was removed *in vacuo* and the residue purified by flash column chromatography (petroleum ether: EtOAc, 6:4) to give **PyrroTriPol-H-OMe** (0.032 g, 0.025 mmol, 86% yield) as a green solid.
TLC (Silica gel, CH₂Cl₂:MeOH 9:1), Rf (**PyrroTriPol-H-OMe**) = 0.4. ESI-HRMS: calcd. for C₅₅H₆₅N₃O₇S₁₂ [M+Na⁺] 1286.1363, measured 1286.1354 (Δm = 0.0009, error = 0.7 ppm).
EPR (ClCH₂CH₂Cl, 1.0 mM): EPR spectrum represented in [Fig.5a].
HPLC: HPLC chromatogram represented in [Fig.5b].

### Synthesis of PyrroTriPol-H

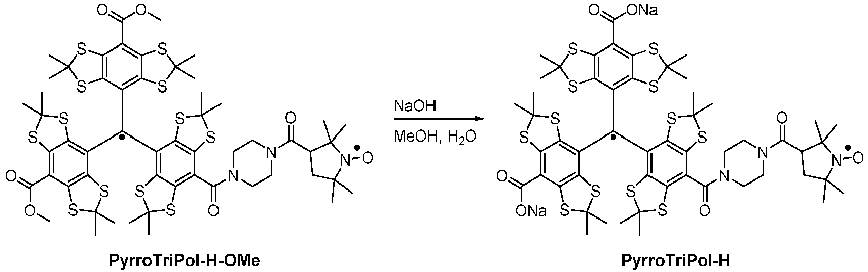

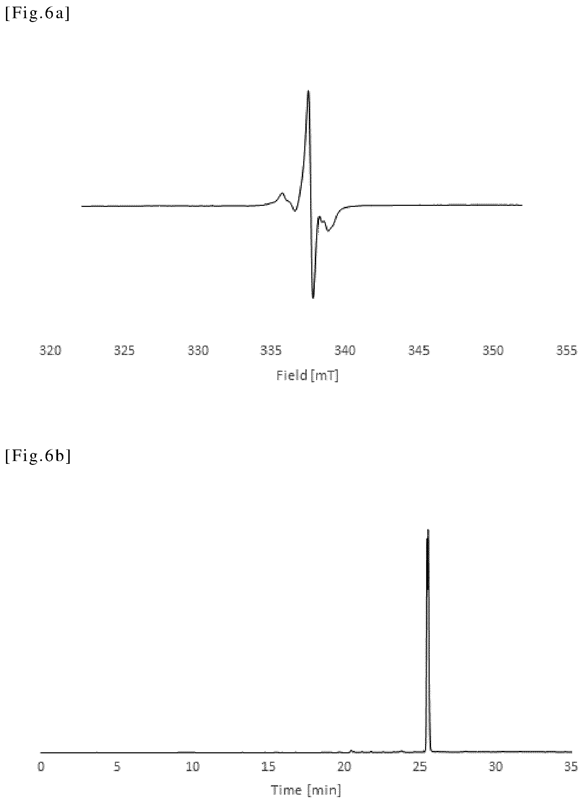

To a solution of **PyrroTriPol-H-OMe** (0.015 g, 0.012 mmol) in MeOH (2.0 mL) was added (0.003 g, 0.072 mmol) and H₂O (0.1 mL). The reaction mixture was stirred at 22 °C for 48 h. The solvent was removed *in vacuo* and the residue purified by C18-HPLC to afford **PyrroTriPol-H** (0.013 g, 0.010 mmol, 88% yield) as a green solid.
ESI-HRMS: calcd. for C₅₃H₆₀N₃O₇S₁₂ [M-H⁺] 1234.1085, measured 1234.1034 (Δm = 0.0051, error = 4.1 ppm).
EPR (H₂O, 1.0 mM): EPR spectrum represented in [Fig.6a].
HPLC: HPLC chromatogram represented in [Fig.6b].

### Example 3:

### Synthesis of DiPyrroTriPol-OMe

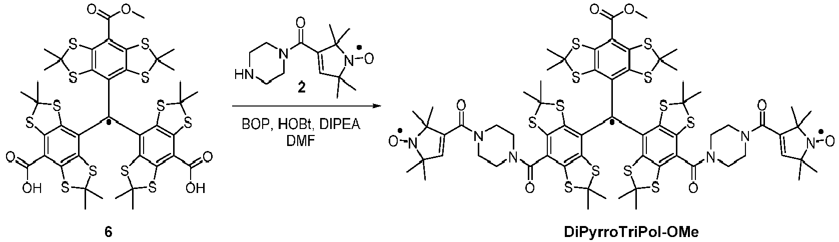

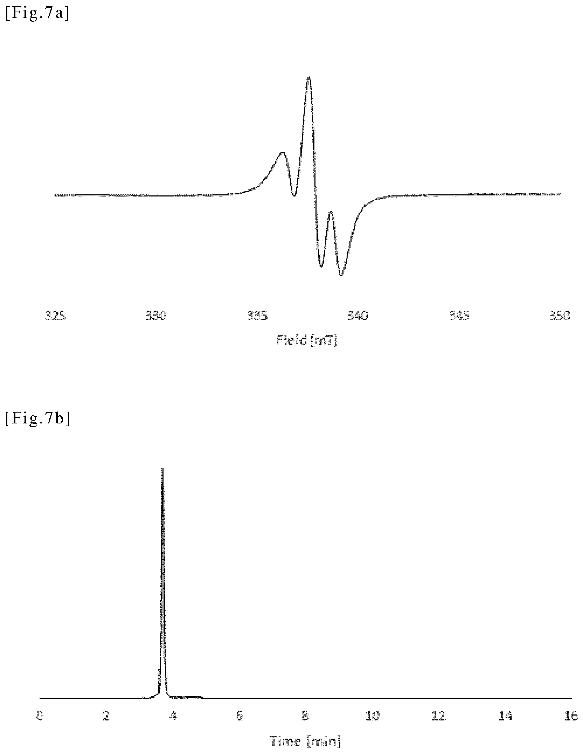

To a solution of trityl **6** (doi: 10.1055/s-0035-1561299) (0.010 g, 0.010 mmol) in DMF (1.0 mL) was added benzotriazol-1-yloxytris(dimethylamino)phosphonium hexafluorophosphate or BOP (0.013 g, 0.030 mmol), *N*-hydroxybenzotriazole or HOBt (0.004 g, 0.030 mmol) and DIPEA (0.011 mL, 0.060 mmol) and the resulting solution was stirred at 22 °C for 5 min. Nitroxide **2** as synthesized in example 1 (0.008 g, 0.030 mmol) was added and the resulting solution was stirred at 22 °C for 12 h. Saturated NaHCO₃ (10 mL) was added and the solution extracted with EtOAc (3×10 mL). The combined organic layers were dried over Na₂SO₄, the solvent was removed *in vacuo* and the residue purified by flash column chromatography (pet. ether: EtOAc, 3:7) to give **DiPyrroTriPol-OMe** (0.013 g,0.009 mmol, 88% yield) as a green solid.
TLC (Silica gel, pet. ether:EtOAc 3:7), Rf (**DiPyrroTriPol-OMe**) = 0.4. ESI-HRMS: calcd. for C₆₇H₈₁N₆O₈S₁₂ [M+Na⁺] 1504.2657, measured 1504.2583 (Δm = 0.0074, error = 4.9 ppm).
EPR (ClCH₂CH₂Cl, 1.0 mM): EPR spectrum represented in [Fig.7a].
HPLC: HPLC chromatogram represented in [Fig.7b].

### Example 4:

### Synthesis of TriPyrroTriPol

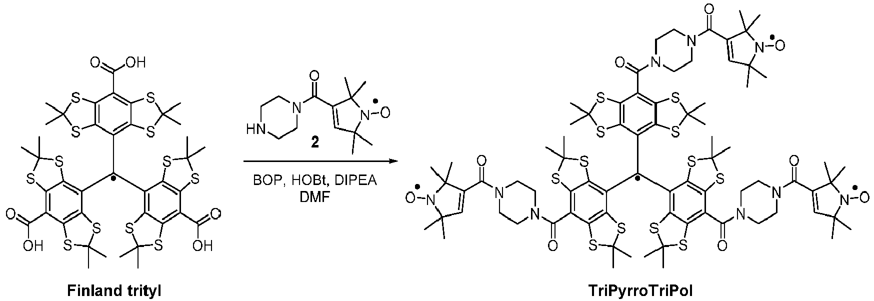

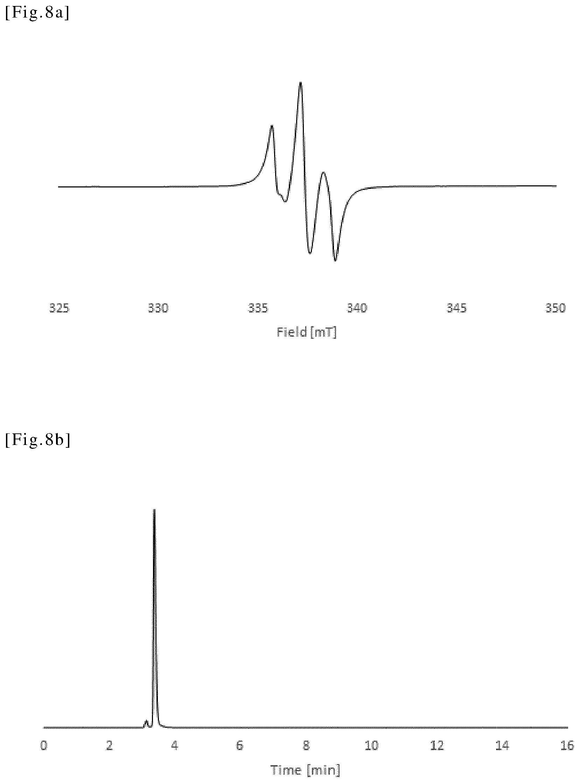

To a solution of **Finland trityl** (doi: 10.1055/s-0035-1561299) (0.010 g, 0.010 mmol) in DMF (1.0 mL) was added benzotriazol-1-yloxytris(dimethylamino)phosphonium hexafluorophosphate or BOP(0.020 g, 0.045 mmol), N-hydroxybenzotriazole or HOBt (0.006 g, 0.045 mmol) and DIPEA (0.016 mL, 0.090 mmol) and the resulting solution was stirred at 22 °C for 5 min. Nitroxide **2** as synthesized in example 1 (0.011 g, 0.045 mmol) was added and the resulting reaction solution was stirred at 22 °C for 12 h. Saturated NaHCO₃(10 mL) was added and the solution extracted with EtOAc (3×10 mL). The combined organic layers were dried over Na₂SO₄, the solvent was removed *in vacuo* and the residue purified by flash column chromatography (pet. ether: EtOAc, 6:4) to give **TriPyrroTriPol** (0.013 g, 0.008 mmol, 76% yield) as a green solid.
TLC (Silica gel, EtOAc), Rf (**TriPyrroTriPol**) = 0.4.
ESI-HRMS: calcd. for C₇₉H₉₉N₉O₉S₁₂Na [M+Na⁺] 1726.4112, measured 1726.4120 (Δm = 0.0008, error = 0.5 ppm).
EPR (ClCH₂CH₂Cl, 1.0 mM): EPR spectrum represented in [Fig.8a].
HPLC: HPLC chromatogram represented in [Fig.8b].

### Example 5:

### Synthesis of DiPyrroTriPol-H-OMe

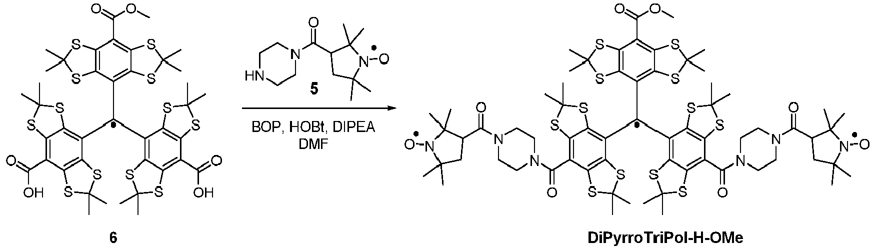

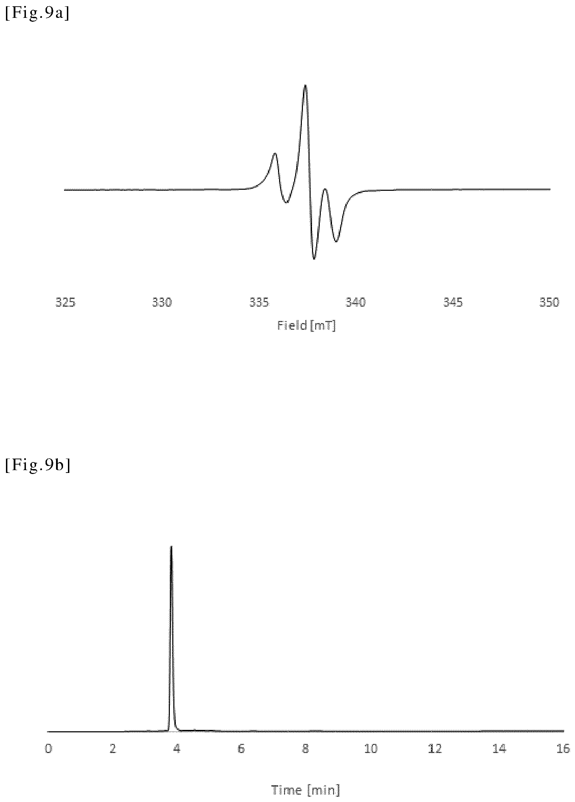

To a solution of trityl **6** (doi: 10.1055/s-0035-1561299) (0.010 g, 0.010 mmol) in DMF (1.0 mL) was added benzotriazol-1-yloxytris(dimethylamino)phosphonium hexafluorophosphate or BOP (0.013 g, 0.030 mmol), *N*-hydroxybenzotriazole or HOBt (0.004 g, 0.030 mmol) and DIPEA (0.011 mL, 0.060 mmol) and the resulting solution was stirred at 22 °C for 5 min. Nitroxide **5** prepared in example 2 (0.008 g, 0.030 mmol) was added and the resulting reaction solution was stirred at 22 °C for 12 h. Saturated NaHCO₃(10 mL) was added and the solution extracted with EtOAc (3×10 mL). The combined organic layers were dried over Na₂SO₄, the solvent was removed *in vacuo* and the residue purified by flash column chromatography (pet. ether: EtOAc, 3:7) to give **DiPyrroTriPol-H-OMe** (0.008 g,0.005 mmol, 54% yield) as a green solid.
TLC (Silica gel, pet. ether:EtOAc 3:7), Rf (**DiPyrroTriPol-H-OMe**) = 0.4. ESI-HRMS: calcd. for C₆₇H₈₅N₆O₈S₁₂ [M+Na⁺] 1508.2970, measured 1508.2953 (Δm = 0.0017, error = 1.1 ppm).
EPR (ClCH₂CH₂Cl, 1.0 mM): EPR spectrum represented in [Fig.9a].
HPLC: HPLC chromatogram represented in [Fig.9b].

### Example 6:

### Synthesis of TriPyrroTriPol-H

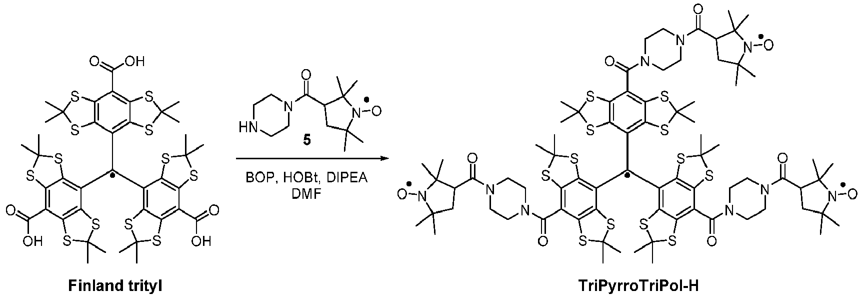

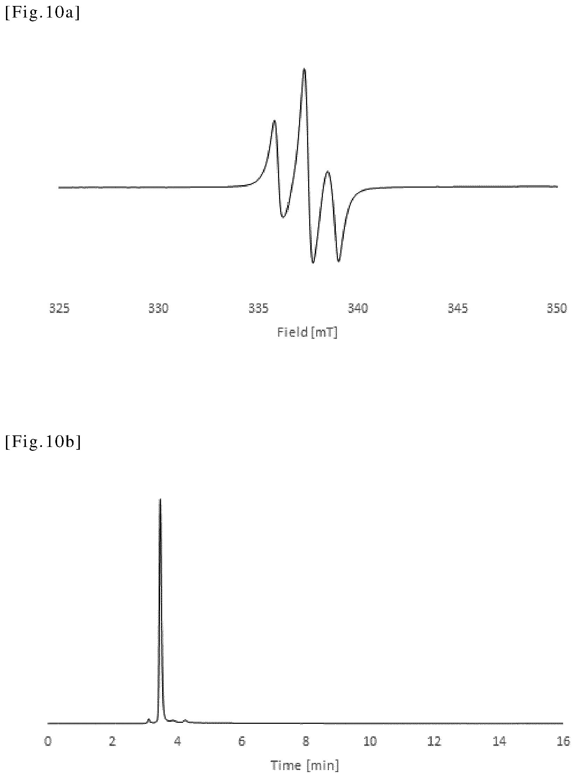

To a solution of **Finland trityl** (doi: 10.1055/s-0035-1561299) (0.010 g, 0.010 mmol) in DMF (1.0 mL) was added benzotriazol-1-yloxytris(dimethylamino)phosphonium hexafluorophosphate or BOP (0.020 g, 0.045 mmol), N-hydroxybenzotriazole or HOBt (0.006 g, 0.045 mmol) and DIPEA (0.016 mL, 0.090 mmol) and the resulting solution was stirred at 22 °C for 5 min. Nitroxide **5**prepared in example 2 (0.011 g, 0.045 mmol) was added and the resulting reaction solution was stirred at 22 °C for 12 h. Saturated NaHCO₃(10 mL) was added and the solution extracted with EtOAc (3×10 mL). The combined organic layers were dried over Na₂SO₄, the solvent was removed *in vacuo* and the residue purified by flash column chromatography (EtOAc) to give **TriPyrroTriPol-H** (0.010 g, 0.006 mmol, 58% yield) as a green solid.
TLC (Silica gel, EtOAc), Rf (**TriPyrroTriPol-H**) = 0.4.
ESI-HRMS: calcd. for C₇₉H₁₀₅N₉O₉S₁₂ [M+Na⁺] 1732.4582, measured 1732.4589 (Δm = 0.0007, error = 0.4 ppm).
EPR (ClCH₂CH₂Cl, 1.0 mM): EPR spectrum represented in [Fig. 10a].
HPLC: HPLC chromatogram represented in [Fig.10b].

The piperazine bridge, linked through amide bonds to trityl and nitroxide moieties as in the above synthesized compounds, increases the interaction between unpaired electrons (called dipolar coupling and exchange interaction) and constrains the relative orientation of the two paramagnetic centers (trityl and nitroxide) in the compounds.

The piperazine bridge/linker has a reduced flexibility and limits the number of accessible conformations of the compounds. This typically translates into a narrower distribution of dipolar couplings and *J*-exchange interactions. In addition, such a rigid bridge/linker also prevents the presence of conformations with too short nitroxide-trityl distances (unlike in the case of TEMTriPol-I), that typically yield a non-optimal DNP transfer.

The above synthesized compounds have been tested successfully. In addition to provide very good polarization transfer efficiency, compared to state-of-the-art polarizing agents, these compounds were still performing well in the fast spinning frequency regime (i.e. MAS frequency larger than 10 kHz) and at high magnetic field (i.e. about 5-21 T). In addition, the newly developed trityl-nitroxides possess an easy synthesis and purification to prepare larger quantities of persistent and efficient trityl-nitroxide biradicals for different solvents.

## Claims

1. A compound of formula (I) 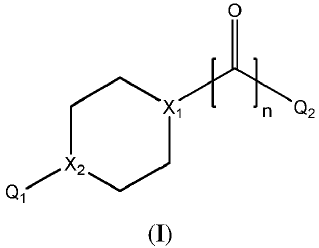 wherein
- n is 0 or 1;
- X₁ and X₂ are, independently, N, P, S;
- Q₂ is 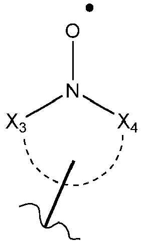 wherein
- X₃ and X₄ are joined, as indicated by 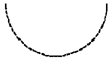 to form together with the nitrogen atom to which they are bound a 5- to 8-membered heterocyclic ring, the heterocyclic ring being optionally substituted, and/or
the heterocyclic ring optionally containing one or more carbon-carbon double bond; 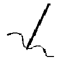 is the point of attachment of the 5- to 8-membered heterocyclic ring to the rest of the molecule;
- Q₁ is selected from 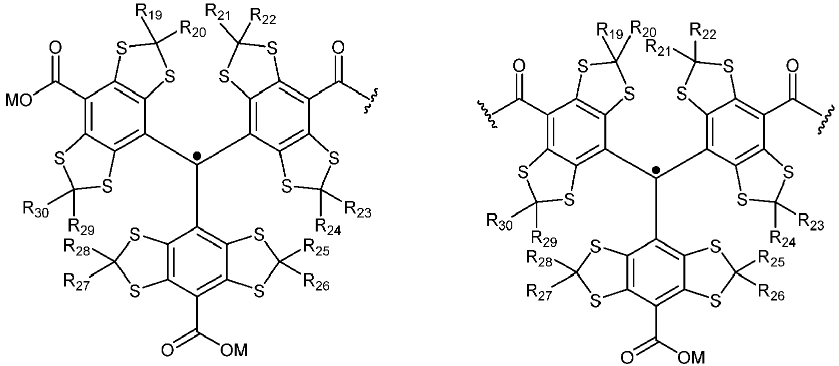 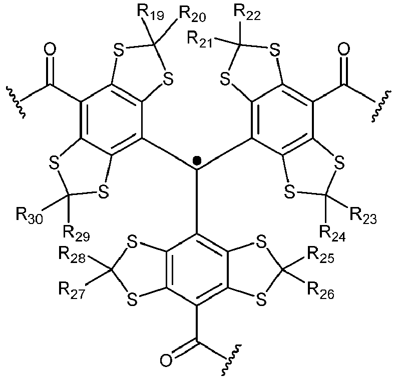 wherein
- R₁₉ to R₃₀ are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms;
- M is a hydrogen, a substituted or unsubstituted linear, branched alkyl having 1 to 10 carbon atoms, a substituted or unsubstituted, a cycloalkyl having 3 to 10 carbon atoms, a substituted or unsubstituted aryl having 6 to 14 carbon atoms, an alkali cation selected from Na⁺ or K⁺, an ammonium cation (NH₄)⁺;
with the proviso that the compound of formula 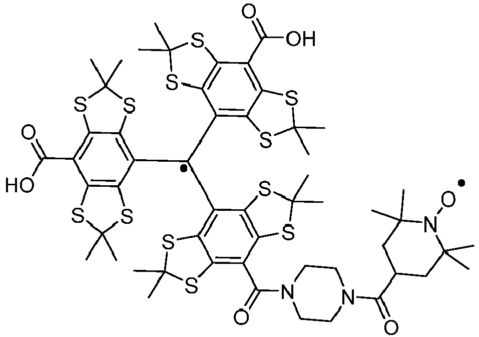 is excluded.

2. The compound of formula (I) according to claim 1, wherein Q₂ is selected from 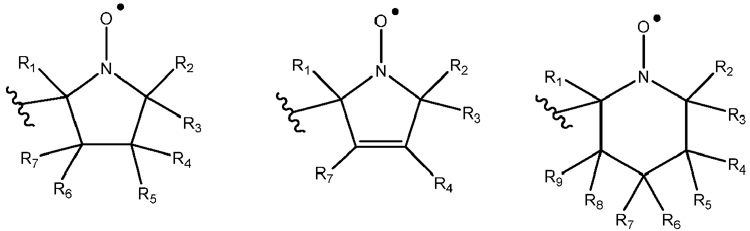 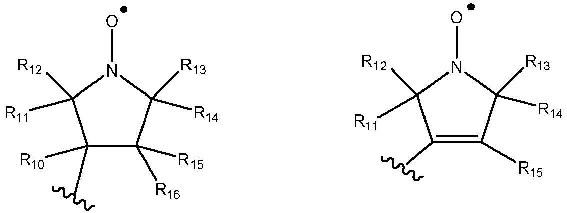 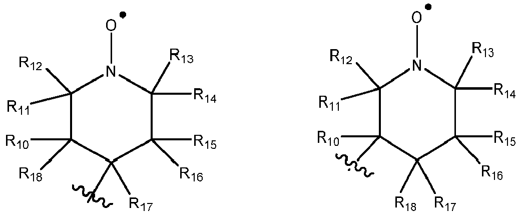 wherein
- R₁ to R₁₈ are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms; or
- R₁ to R₉, R₁₀, and R₁₅ to R₁₈, are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms, and
geminal R₁₁ and R₁₂, and geminal R₁₃ and R₁₄, are joined to form together with the secondary carbon to which they are bound a substituted or unsubstituted cycloalkyl having 3 to 10 carbon atoms, a substituted or unsubstituted 5- or 6-membered heterocyclic ring wherein at least one ring atom is oxygen; 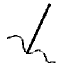 is the point of attachment of the 5- to 6-membered heterocyclic ring to the rest of the molecule.

3. The compound of formula (I) according to claim 1 or 2, wherein Q₂ is selected from 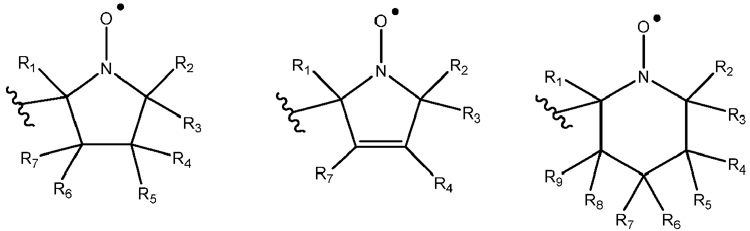 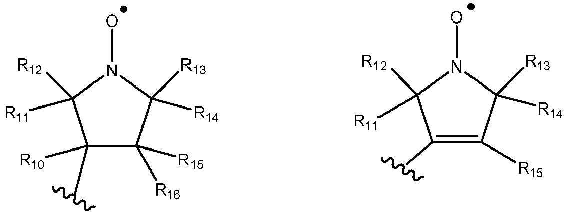 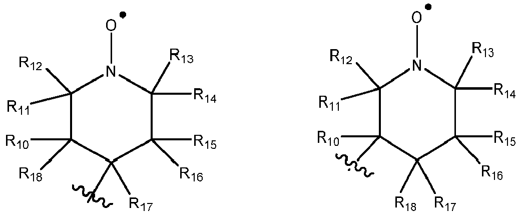
wherein R₁ to R₁₈ are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms; 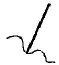
is the point of attachment of the 5- to 6-membered heterocyclic ring to the rest of the molecule.

4. The compound of formula (I) according to claim 3, wherein R₁ to R₁₈ are, independently, hydrogen, a substituted or unsubstituted linear, branched alkyl having 1 to 6 carbon atoms, a substituted or unsubstituted cycloalkyl having 3 to 6 carbon atoms, a substituted or unsubstituted aryl having 6 to 14 carbon atoms.

5. The compound of formula (I) according to any one of claims 1 to 4, wherein Q₂ is selected from 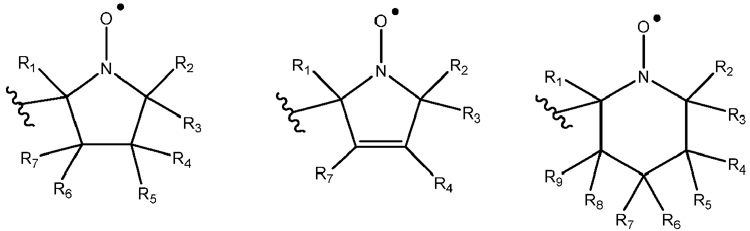 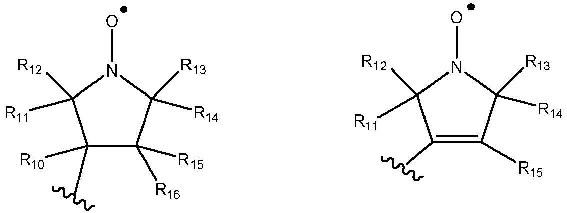 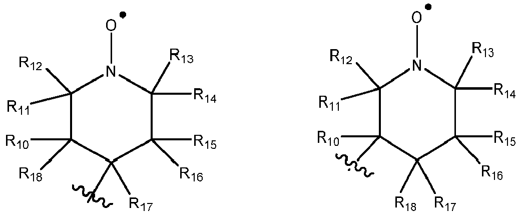 wherein
- R₁ to R₉, R₁₀, and R₁₅ to R₁₈, are, independently, hydrogen, a substituted or unsubstituted linear, branched alkyl having 1 to 10 carbon atoms, a substituted or unsubstituted cycloalkyl having 3 to 10 carbon atoms, a substituted or unsubstituted aryl having 6 to 14 carbon atoms, and
geminal R₁₁ and R₁₂, and geminal R₁₃ and R₁₄, are joined to form together with the secondary carbon to which they are bound a substituted or unsubstituted cycloalkyl having 3 to 10 carbon atoms, a substituted or unsubstituted 5- or 6-membered heterocyclic ring wherein at least one ring atom is oxygen; 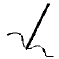
is the point of attachment of the 5- to 6-membered heterocyclic ring to the rest of the molecule.

6. The compound of formula (I) according to claim 5, wherein
- R₁ to R₉, R₁₀, and R₁₅ to R₁₈, are, independently, hydrogen, a substituted or unsubstituted linear, branched alkyl having 1 to 6 carbon atoms, and
geminal R₁₁ and R₁₂, and geminal R₁₃ and R₁₄, are joined to form together with the secondary carbon to which they are bound a substituted or unsubstituted cycloalkyl having 3 to 6 carbon atoms, a substituted or unsubstituted 5- or 6-membered heterocyclic ring with one ring atom being oxygen.

7. The compound of formula (I) according to any one of claims 1 to 6, wherein Q₂ is selected from 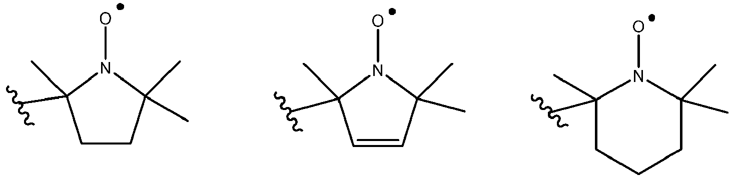 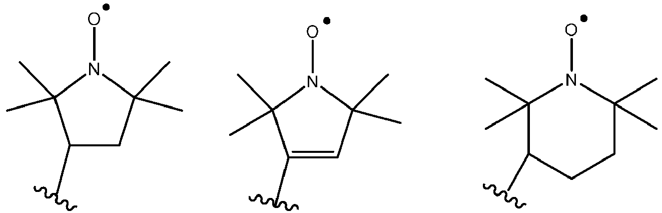 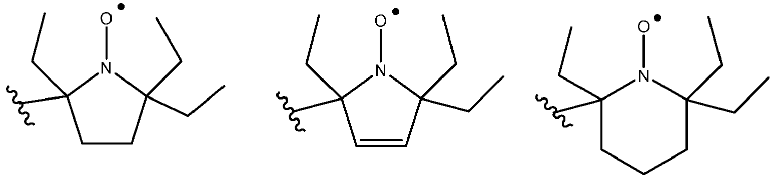 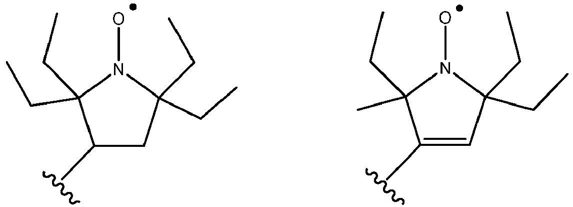 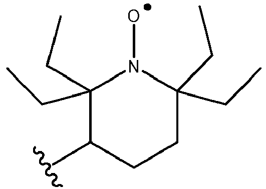 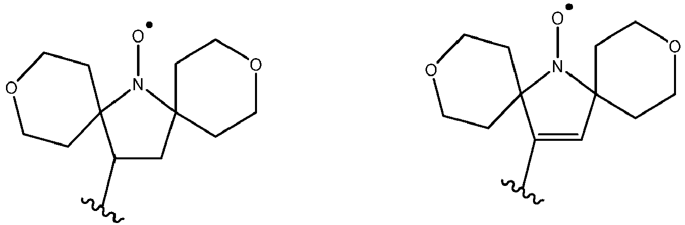 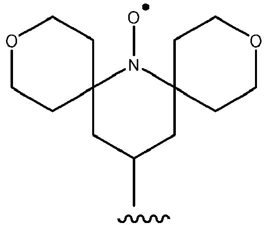 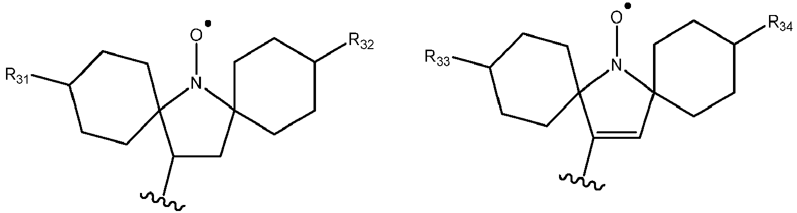 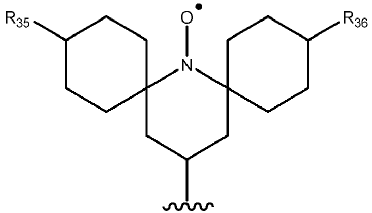 with R₃₁ to R₃₆, independently, representing
• a hydrogen;
• a hydroxyl;
• an alkoxy with the alkyl being methyl, ethyl, propyl, butyl, pentyl, hexyl, and their branched isomers;
• an unsubstituted alkyl selected from methyl, ethyl, propyl, butyl, pentyl, hexyl, and their branched isomers;
• a substituted alkyl selected from methyl, ethyl, propyl, butyl, pentyl, hexyl, and their branched isomers, the substitution including CO₂Mₐ with Ma being an alkali metal selected in the group consisting of sodium (Na), potassium (K); -NRaRbRc with Rₐ, R_{b} and R_{c} being, independently, methyl, ethyl, propyl, butyl, pentyl, hexyl, and their branched isomers; -OPO₃(M_{b})₂ with Mb being a hydrogen, an alkali metal selected in the group consisting of sodium (Na), potassium (K);
• CO₂Mₐ with Ma being an alkali metal selected in the group consisting of sodium (Na), potassium (K);
• -NRaRbRc with Rₐ, R_{b} and R_{c} being, independently, methyl, ethyl, propyl, butyl, pentyl, hexyl, and their branched isomers;
• -OPO₃(M_{b})₂ with Mb being a hydrogen, an alkali metal selected in the group consisting of sodium (Na), potassium (K); 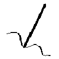
is the point of attachment of the 5- to 6-membered heterocyclic ring to the rest of the molecule.

8. The compound of formula (I) according to any one of claims 1 to 7, wherein Q₁ is selected from 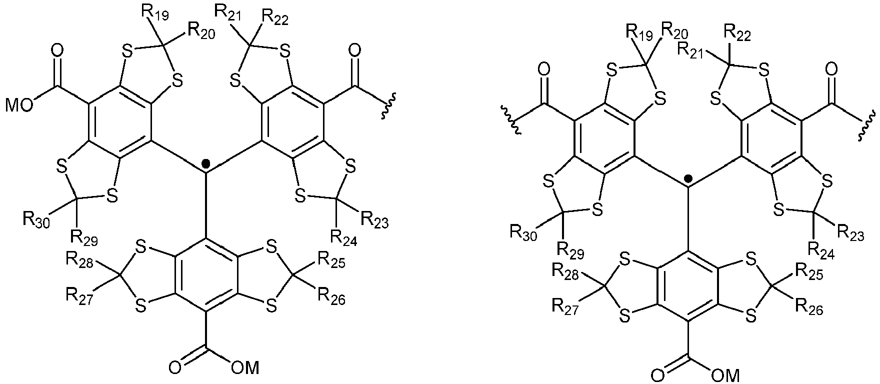 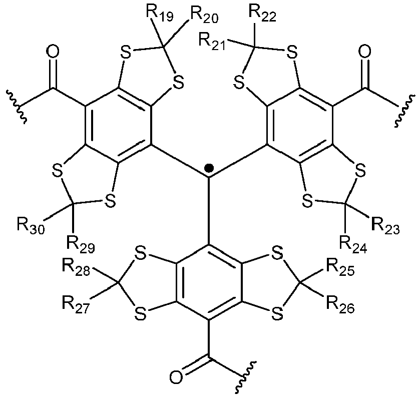 wherein
- R₁₉ to R₃₀ are, independently, hydrogen, a substituted or unsubstituted linear, branched alkyl having 1 to 6 carbon atoms, a substituted or unsubstituted cycloalkyl having 3 to 6 carbon atoms, a substituted or unsubstituted aryl having 6 to 14 carbon atoms;
- M is a hydrogen, a substituted or unsubstituted linear, branched alkyl having 1 to 6 carbon atoms, a substituted or unsubstituted cycloalkyl having 3 to 6 carbon atoms, a substituted or unsubstituted aryl having 6 to 14 carbon atoms, an alkali cation selected from Na⁺ or K⁺, an ammonium cation (NH₄)⁺; 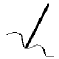
is the point of attachment of the ―(C=O) group to the rest of the molecule.

9. The compound of formula (I) according to any one of claims 1 to 8, wherein Q₁ is selected from 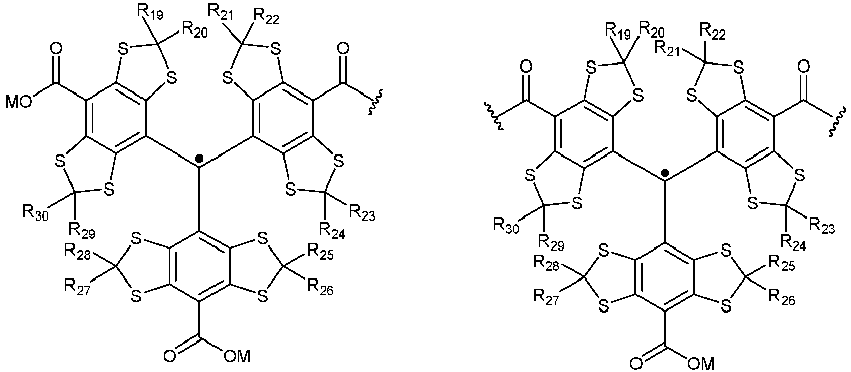 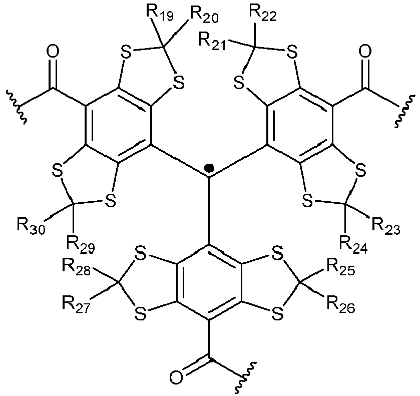 wherein
- R₁₉ to R₃₀ are, independently, hydrogen, a substituted or unsubstituted linear, branched alkyl having 1 to 6 carbon atoms;
- M is a hydrogen, a substituted or unsubstituted linear, branched alkyl having 1 to 6 carbon atoms, an alkali cation selected from Na⁺ or K⁺, an ammonium cation (NH₄)⁺; 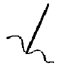 is the point of attachment of the ―(C=O) group(s) to the rest of the molecule.

10. The compound of formula (I) according to any one of claims 1 to 9, wherein Q₁ is selected from 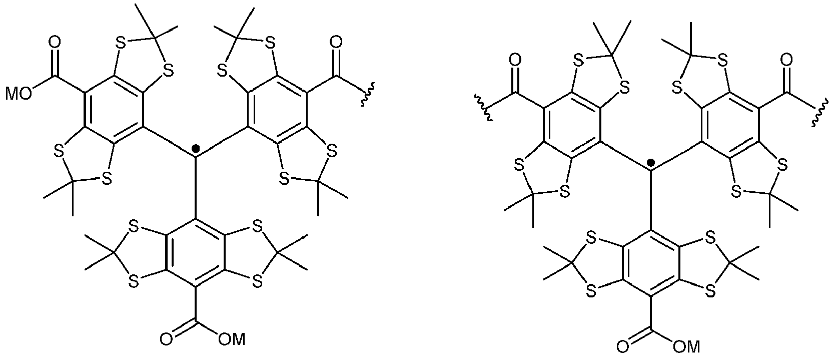 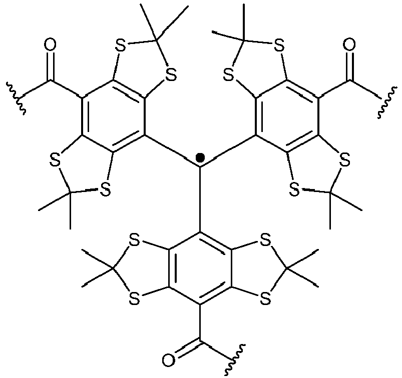
wherein M is a hydrogen, an unsubstituted alkyl selected from methyl, ethyl, propyl, butyl, pentyl, hexyl, and their branched isomers, an alkali cation selected from Na⁺ or K⁺, an ammonium cation (NH₄)⁺; 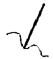
is the point of attachment of the ―(C=O) group(s) to the rest of the molecule.

11. The compound of formula (I) according to any one of claims 1 to 10, wherein X₁ and X₂ are N.

12. The compound of formula (I) according to any one of claims 1 to 11, wherein it is selected from: 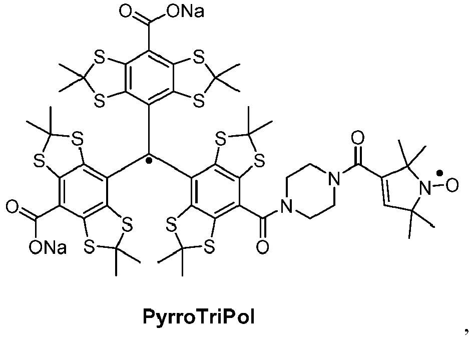 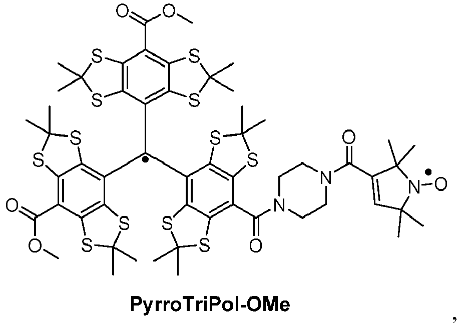 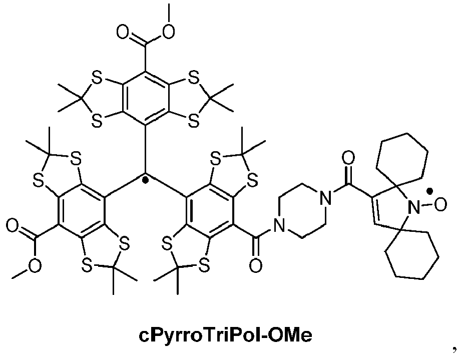 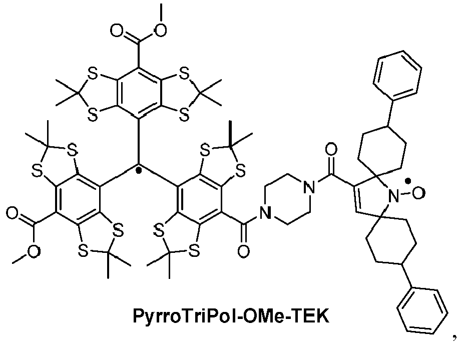 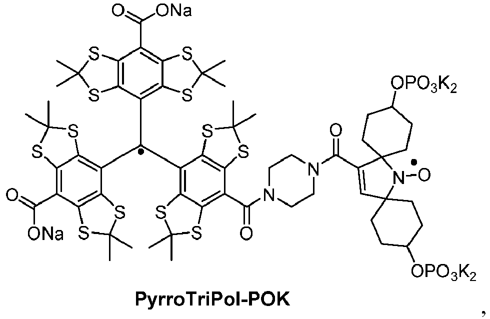 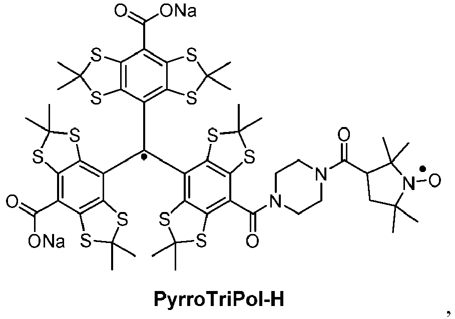 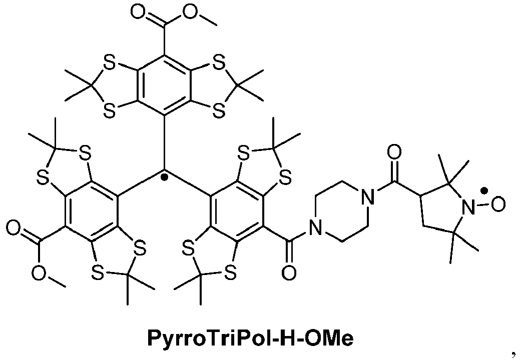 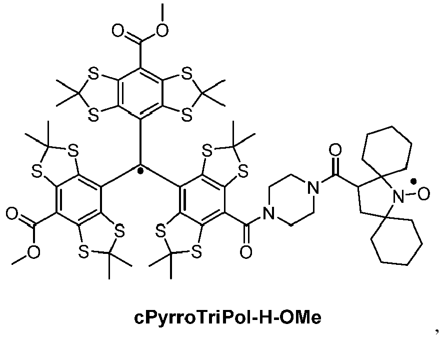 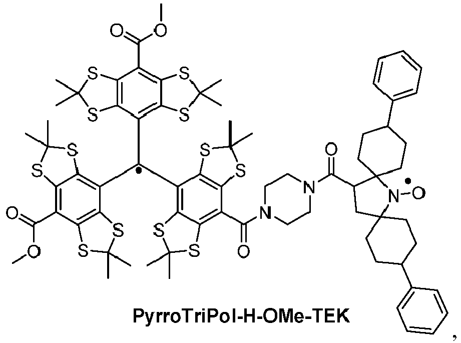 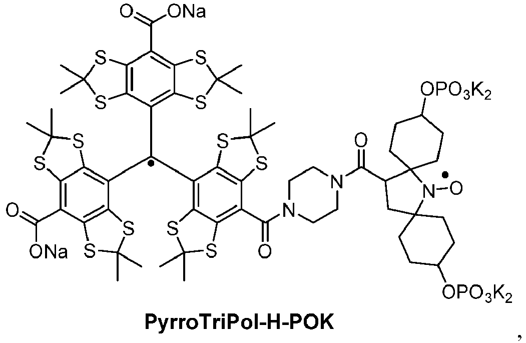 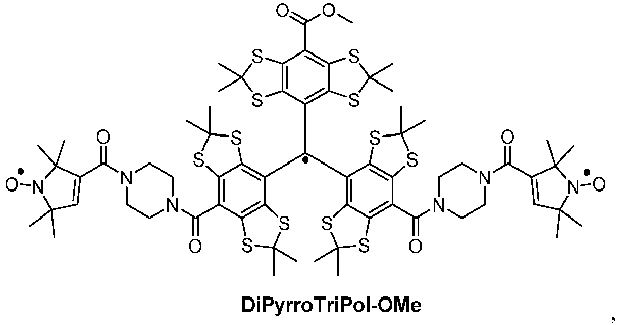 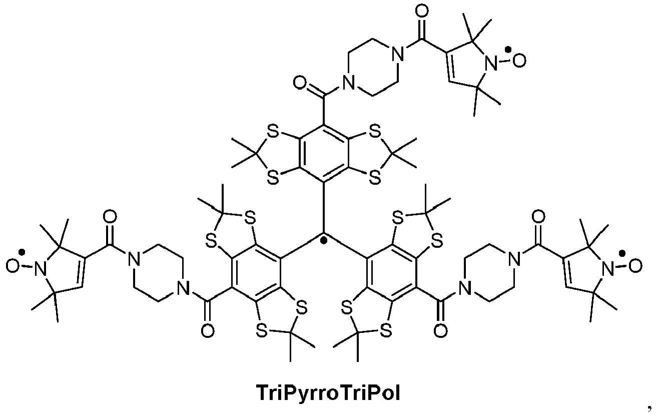 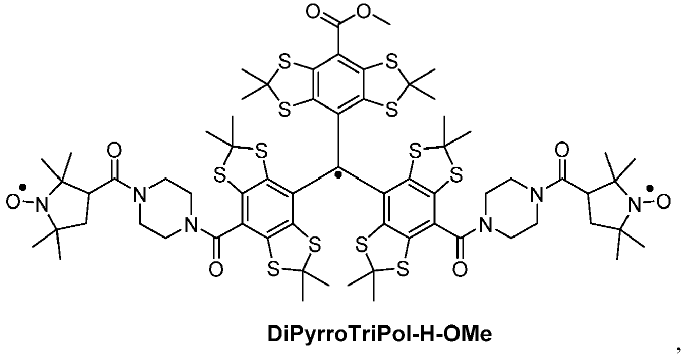 and

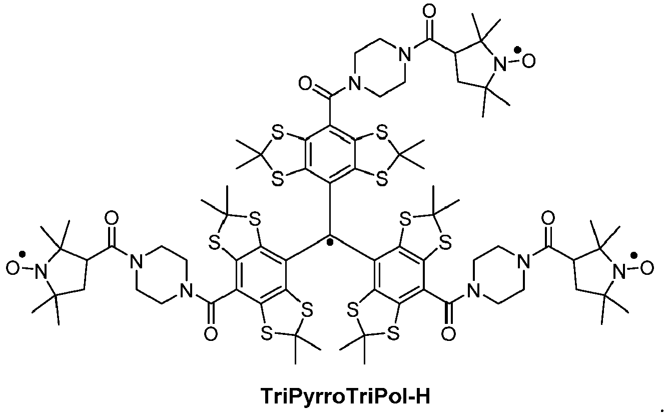

13. The use of a compound of formula (I) according to any one of claims 1 to 12, as a polarizing agent.

14. The use of a compound of formula (I) according to any one of claims 1 to 12, as a polarizing agent (PA) for DNP in the context of structural biology, material sciences, Nuclear Magnetic Resonance of solids or applied to liquid samples, particle physics, and medical imaging.

15. A method for polarizing an analyte in a sample for Dynamic Nuclear Polarization comprising the steps of
a) providing a sample comprising an analyte;
b) contacting said sample with a compound of formula (I) according to any one of claims 1 to 12 as polarizing agent (PA) that enables an optimal nuclear polarization of the analyte in a magnetic field;
c) irradiating said sample with at least one radiation that causes electron spin flip, to enhance the performance of NMR detection or MRI performance; and
d) optionally dissolving the sample and obtaining a hyperpolarized sample;
said method optionally further comprises observing the NMR or MRI of the hyperpolarized sample.
